# FASCICULE DE BREVET EUROPEEN

(11) **EP 3 844 144 B1**
(45) Date de publication et mention de la délivrance du brevet: **13.12.2023**
(21) Numéro de dépôt: 19774158.0
(22) Date de dépôt: 30.08.2019
(51) Int. Cl.: C07C 245/22, C07C 247/04, C07C 255/26, C07F 9/00

(54) **NOUVEAUX COMPOSES TETRAZENES FONCTIONNALISES**
NEUE FUNKTIONALISIERTE TETRAZENVERBINDUNGEN
NEW FUNCTIONALISED TETRAZENE COMPOUNDS

(30) Priorité: 31.08.2018 FR 1857854
(43) Date de publication de la demande: 07.07.2021
(73) Titulaire: ArianeGroup SAS, 78130 Les Mureaux (FR); Centre national de la recherche scientifique, 75016 Paris (FR); Université Claude Bernard (Lyon I), 69100 Villeurbanne cedex (FR)
(72) Inventeur: JOUCLA, Lionel, 69300 CALUIRE ET CUIRE (FR); DHENAIN, Anne, 01330 VILLARS-LES-DOMBES (FR); DARWICH, Chaza, 69006 LYON (FR); EYMANN, John, 69100 VILLEURBANNE (FR); JACOB, Guy, 91710 VERT LE PETIT (FR)
(74) Mandataire: Cabinet Beau de Loménie
(86) Numéro de dépôt international: PCT/FR2019/052005
(87) Numéro de publication internationale: WO 2020/043997

(56) Documents cités:
- BERND PORATH ET AL: "Hydrogen Bonding of 2-Tetrazenes, 2. Synthesis and Structural Studies of Hydroxyalkyl-Substituted 2-Tetrazenes", ZEITSCHRIFT FUR NATURFORSCHUNG - SECTION B JOURNAL OF CHEMICAL SCIENCES, vol. 57, no. 4, 1 avril 2002 (2002-04-01), pages 365-376, XP055603537, DE ISSN: 0932-0776, DOI: 10.1515/znb-2002-0402 cité dans la demande
- NELSEN, S.F. ET AL: "Effect of benzyl groups on electron loss from tetralakylhydrazines and 2-tetrazenes", J.ORG.CHEM., vol. 46, 1981, pages 2402-2404, XP002792925,

## Description

La présente invention a pour principal objet des tétrazènes (di-)fonctionnalisés, plus précisément des 1,4-diméthyltétrazènes (di-)fonctionnalisés en position β, en position γ ou en positions β et γ. Elle a également pour objet la préparation et les utilisations desdits tétrazènes (di-)fonctionnalisés.

Ces composés chimiques peuvent être utilisés, de manière générale, en synthèse organique, en particulier pour des applications en chimie fine, dans les industries pharmaceutiques et cosmétiques. Certains (au vu de leur faible teneur en carbone) sont des candidats intéressants pour la propulsion, notamment la propulsion hybride (ils peuvent être considérés comme des substituts de l'hydrazine) et/ou des candidats intéressants pour remplacer les esters nitriques présents, à titre de plastifiant, dans les propergols solides.

Selon l'art antérieur, des tétrazènes fonctionnalisés ont déjà été décrits, notamment ceux présentant les formules I à III ci-après :

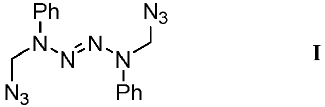

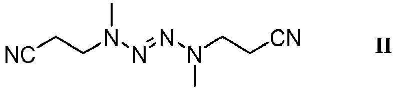

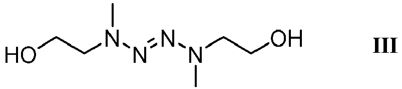

Le tétrazène de formule I, fonctionnalisé par des groupes N₃, a été décrit dans le J. Chem. Soc. Chem. Commun. 1974, 264-265. Il présente l'inconvénient de renfermer une part carbonée très importante, de par la présence des groupes phényle. On comprend, dans cet esprit, que les tétrazènes de l'invention sont opportunément des 1,4-diméthyltétrazènes (voir ci-après).

Le 1,4-diméthyltétrazène de formule **II,** fonctionnalisé par des groupes CN, a été décrit, ainsi que sa synthèse, dans le Bull. Russ. Acad. Sci. Div. Chem. Sci. 1992, 41, 1400-1413.

Le 1,4-diméthyltétrazène de formule **III,** fonctionnalisé par des groupes OH, a été décrit dans le Zeitschrift fur Naturforschung, B: Chemical Sciences 2002, 57 (4), 365-376 (son homologue tétra-hydroxylé a aussi été décrit par les mêmes auteurs). La voie de synthèse décrite est reproduite sur le schéma ci-après :

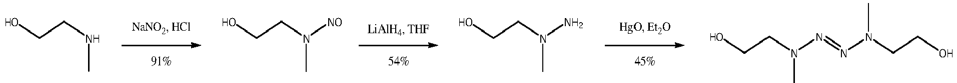

Elle est complexe (3 étapes) et présente de nombreux inconvénients : le dérivé N-nitroso intermédiaire (obtenu à partir de la N-méthyl-N-hydroxyéthyl amine) est hautement cancérigène, l'hydrure utilisé est extrêmement réactif, voire dangereux, l'utilisation de l'oxydant à base de mercure pose évidemment problème et enfin, conjointement à l'oxydant à base de mercure, un solvant éthéré (dont l'inflammabilité, la volatilité et la présence potentielle de peroxydes en son sein contraignent fortement l'utilisation à l'échelle industrielle) est utilisé. Il est du mérite des inventeurs de proposer une voie de synthèse beaucoup plus intéressante pour ce tétrazène de formule **III** et son homologue γ-substitué (voir ci-après) et de s'être intéressés à des éthers de ceux-ci.

Nelsen et al. (J. Org. Chem. 1981, 46, 2402-2404) ont étudié l'influence de groupes benzyles sur la perte électronique de tétraalkylhydrazines et de 2-tétrazènes.

Dans un tel contexte, la Demanderesse propose donc de nouveaux 1,4-diméthyltétrazènes qui sont (di-)fonctionnalisés en position β, en position γ ou en positions β et γ. Ils répondent respectivement à la formule **(Ia),** à la formule **(Ib)** ou à la formule (Ic) ci-après :

**R1-CH₂-N(CH₃)-N=N-N(CH₃)-CH₂-R2** **(Ia)**

**R3-CH₂-CH₂-N(CH₃)-N=N-N(CH₃)-CH₂-CH₂-R4** **(Ib)**

**R5-CHOH-CH₂-N(CH₃)-N=N-N(CH₃)-CH₂-CHOH-R5** **(Ic)**

Ils répondent plus précisément :
- à ladite formule **(Ia),** dans laquelle :
   R1 = R2 = **-CH₂Cl, -CH₂N₃, -CH₂NH₂, -CH₂N(CH₃)(NH₂), -CH=CH₂,** ou **-CH₂NCO;** ou
   R1 et R2, identiques, renfermant un groupe éther (obtenu par alkylation des deux fonctions -CH2OH du tétrazène de formule III (voir ci-après)), sont choisis parmi les couples ci-après :
   R1 = R2 = **-CH₂O-CH₂-CH=CH₂,**
   R1 = R2 = **-CH₂O-CH₂-C≡CH,** et
   R1 = R2 **=-CH₂O-CH₂-C≡N ;**
- à ladite formule **(Ib)**, dans laquelle :
   R3 = R4 = **-CH₂OH, -CH₂N₃, -CH₂NH₂, -CH₂N(CH₃)(NH₂), -CH=CH₂, -CH₂NCO, -CH₂CN ou -CH₂ONO₂;** ou
   R3 = **-CH₂OH** et R4 = **-CH₂N₃,** ou
   R3 = **-CH₂CN** et R4 = **-CH₂N₃,** ou
   R3 = **-CH₂N₃** et R4 = **-CH₂N(CH₃)(NH₂),** ou
   R3 et R4, identiques ou différents, l'un au moins d'entre eux renfermant un groupe éther (obtenu par alkylation d'au moins une fonction -CH2OH), sont choisis parmi les couples ci-après :
      R3 = R4 = **-CH₂O-CH₂-CH=CH₂**
      R3 = R4 = **-CH₂O-CH₂-C≡CH**
      R3 = R4 = **-CH₂O-CH₂-C≡N**
      R3 = **-CH₂O-CH₂-CH=CH₂** et R4 = **-CH₂N₃**
      R3 = **-CH₂O-CH₂-C≡CH** et R4 = **-CH₂N₃,** et
      R3 = **-CH₂O-CH₂-C≡N** et R4 = **-CH₂N₃** ; ou
- à ladite formule **(Ic)**, dans laquelle :
   R5 = **-CH₂N₃** ou **-CH₂CN.**

Pour ce qui concerne les tétrazènes de formule **(Ia)** et **(Ib),** on a compris qu'en référence, respectivement, aux valeurs de R1 et R2 et aux valeurs de R3 et R4, ils peuvent être répartis en deux groupes, le second desdits deux groupes correspondant à des tétrazènes avec, respectivement, lesdites valeurs R1 et R2 et au moins une desdites valeurs R3 et R4, renfermant un groupe éther (de type éther d'allyle, éther de propargyle ou éther d'acétonitrile). De tels tétrazènes, di-fonctionnalisés, respectivement en position β et en position γ, sont obtenus à partir du tétrazène correspondant di-fonctionnalisé en position β par deux fonctions hydroxy et mono- ou di-fonctionnalisé en position γ par une ou deux fonctions hydroxy. Le tétrazène di-fonctionnalisé en position β par des fonctions hydroxy (« de formule (Ia) avec R1 = R2 = -CH₂OH ») ne fait pas partie de l'invention dans la mesure où il s'agit du tétrazène de l'art antérieur répondant à la formule **III** reproduite ci-dessus.

On peut d'ores et déjà indiquer ici que pour l'obtention desdits tétrazènes (di-)fonctionnalisés par une fonction hydroxy (-OH) en position β (tétrazène de l'art antérieur) et en position γ (tétrazène nouveau présentement revendiqué), les inventeurs proposent une voie de synthèse particulièrement avantageuse au regard de celle décrite dans l'art antérieur pour l'obtention dudit tétrazène (di-)fonctionnalisé par une fonction hydroxy en position β (dont les trois étapes ont été rappelées ci-dessus).

La voie de synthèse proposée par les inventeurs comprend :
- la mise à disposition d'une hydrazine précurseur choisie parmi la 1-(2-hydroxyéthyl)-1-méthylhydrazine et la 1-(3-hydroxypropyl)-1-méthylhydrazine (ou 3-(1-méthylhydrazinyl)propan-1-ol), puis
- par oxydation, la dimérisation de celle-ci.

La 1-(2-hydroxyéthyl)-1-méthylhydrazine est commercialement disponible auprès de la société Aldrich ; la 1-(3-hydroxypropyl)-1-méthylhydrazine est commercialement disponible auprès de la société Ambinter Stock Screening Collection (son coût est élevé). Selon une variante de mise en oeuvre, les inventeurs préconisent la préparation amont desdites hydrazines précurseurs (en une seule étape) : par condensation d'un excès de monométhylhydrazine (MMH) sur, respectivement, le 2-chloroéthanol et le 3-chloropropanol (ladite condensation étant mise en oeuvre sous gaz inerte avec contrôle de la température (des précisions sont fournies plus loin sur cette étape de condensation).

L'oxydation (des hydrazines commerciales ou préparées selon le mode opératoire préconisé par les inventeurs (voir ci-dessus)) peut en fait être mise en oeuvre selon différents modes opératoires et tout particulièrement a) avec le diiode (I₂) en solution dans un solvant organique, notamment choisi parmi l'éthanol et l'éther diéthylique et consistant avantageusement en l'éthanol ou b) avec KI₃ en solution aqueuse ou encore c) avec la monochloramine. Selon des variantes avantageuses :
- la 1-(2-hydroxyéthyl)-1-méthylhydrazine est oxydée avec le diiode (I₂) en solution dans l'éthanol, et
- la 1-(3-hydroxypropyl)-1-méthylhydrazine est oxydée avec KI₃ en solution aqueuse ou avec le diiode (I₂) en solution dans l'éthanol, très avantageusement avec le diiode (I₂) en solution dans l'éthanol.

Ainsi, la voie de synthèse proposée par les inventeurs peut-elle avantageusement comprendre les deux étapes schématisées ci-après :

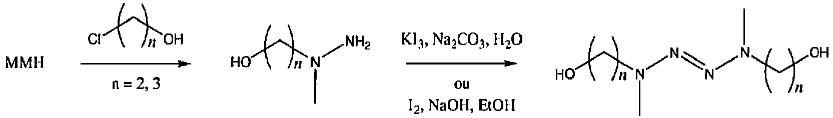

La mise en oeuvre de ces deux étapes successives est illustrée dans les exemples 1 et 1' ci-après.

Cette voie de synthèse en une ou deux étapes (selon que la mise à disposition de l'hydrazine précurseur se résume à la fourniture de ladite hydrazine, commercialement disponible, ou consiste en la synthèse de celle-ci) ne présente pas les inconvénients de celle décrite dans l'art antérieur et assure des rendements intéressants.

Cette voie de synthèse, originale, pour l'obtention du tétrazène de formule **III** de l'art antérieur constitue un objet de l'invention.

Cette voie de synthèse pour l'obtention du tétrazène de formule **(Ib)** dans laquelle R3 = R4 = **-CH₂OH** est à nouveau décrite plus avant dans le présent texte, lors de la description du procédé de préparation des tétrazènes de formule **(Ib).**

Pour l'obtention des tétrazènes de l'invention, de formule **(Ia), (Ib)** et **(Ic),** on préconise de procéder comme indiqué ci-après. Le procédé (général) en cause (qui constitue le deuxième objet de la présente invention) comprend les deux étapes clés ci-après : mise à disposition d'une hydrazine (précurseur) convenable (connue et/ou pouvant être préparée en utilisant de la monométhylhydrazine (MMH) : des conditions d'obtention des hydrazines sont précisées ci-dessus et ci-dessous et sont également largement décrites dans les exemples) et dimérisation de celle-ci par oxydation (des conditions possibles de mise en oeuvre de ladite dimérisation (a) avec le diiode en solution dans un solvant organique, notamment choisi parmi l'éthanol et l'éther diéthylique et consistant avantageusement en l'éthanol ou b) avec KI₃ en solution aqueuse ou c) avec la monochloramine) sont précisées ci-dessus et ci-dessous et sont également largement décrites dans les exemples). Ledit procédé général se décline selon de nombreuses variantes, essentiellement au vu du mode exact de mise à disposition de l'hydrazine précurseur et de possibles diverses réactions supplémentaires pour l'obtention de tétrazènes de l'invention de génération ultérieure.

Pour l'obtention des 1,4-diméthyltétrazènes, (di-)fonctionnalisés en position β, de formule **(Ia),** on propose un procédé de préparation qui comprend :
- la mise à disposition d'une hydrazine précurseur choisie parmi la 1-(2-chloroéthyl)-1-méthylhydrazine (sous forme libre), le chlorhydrate de ladite 1-(2-chloroéthyl)-1-méthylhydrazine ou un de leurs mélanges, la 1-(2-azidoéthyl)-1-méthylhydrazine, la 1-(2-aminoéthyl)-1-méthylhydrazine, et la 1-allyl-1-méthylhydrazine, puis, par oxydation, l'obtention du dimère correspondant, tétrazène de formule **(Ia)** dans laquelle, respectivement, R1 = R2 = **-CH₂Cl, -CH₂N₃, -CH₂NH₂** ou **-CH=CH₂;** ou
- l'obtention du *(*E*)*-1,4-bis(2-hydroxyéthyl)-1,4-diméthyltétrazène (ou *(E)-*3,3'-(1,4-diméthyltétraaz-2-ène-1,4-diyl)bis(éthan-1-ol)) et l'alkylation de ses (deux) fonctions hydroxyle (-OH) pour l'obtention des di-éthers symétriques (R1 = R2 = voir ci-dessus) ; et
- possiblement, la conversion, par substitution nucléophile avec de la monométhylhydrazine, du tétrazène de formule **(Ia)** dans laquelle R1 = R2 = **-CH₂Cl** en le tétrazène de formule **(Ia)** dans laquelle R1 = R2 = **-CH₂N(CH₃)(NH₂)** ou la conversion du tétrazène de formule **(Ia)** dans laquelle R1 = R2 = **-CH₂NH₂,** avantageusement par réaction de ses fonctions amine (-NH₂) avec du 1,1'-carbonyldiimidazole, en le tétrazène de formule **(Ia)** dans laquelle R1 = R2 = **-CH₂NCO.** Pour ce qui concerne la mise à disposition de l'hydrazine précurseur, on peut indiquer ce qui suit. L'hydrazine en cause peut consister en un produit connu, voire commercialisé. Il peut s'agir d'un produit nouveau. En tout état de cause, ladite hydrazine peut être obtenue à partir de la monométhylhydrazine (MMH). Ainsi :
- le chlorhydrate de 1-(2-chloroéthyl)-1-méthylhydrazine : ce composé est connu, il est commercialement disponible. Il peut être obtenu par condensation d'un excès de 1,2-dichloroéthane (DCE) sur la monométhylhydrazine (MMH). La réaction a été décrite, en 1973, par Böhme et al. dans Arch. Pharmaz. 307/74, 272-277. Sa mise en oeuvre est illustrée dans la première partie de l'exemple 2. La 1-(2-chloroéthyl)-1-méthylhydrazine (sous forme libre) peut être directement utilisée. Elle est aussi commercialement disponible. En fait, l'hydrazine précurseur peut être utilisée sous forme chlorhydratée (susceptible de générer la forme libre *in situ* (voir ci-après)) et/ou directement sous forme libre (la forme libre étant la forme active) ;
- la 1-(2-azidoéthyl)-1-méthylhydrazine : à la connaissance des inventeurs, ce composé est nouveau. Il est avantageusement obtenu par azidation de la 1-(2-chloroéthyl)-1-méthylhydrazine, ci-dessus mentionnée (l'azidation peut être mise en oeuvre directement sur l'hydrazine libre ou sur de l'hydrazine libre obtenue à partir du chlorhydrate de ladite hydrazine). Pour l'obtention de la forme libre à partir du chlorhydrate, on peut procéder ainsi : une solution aqueuse dudit chlorhydrate est constituée, refroidie puis neutralisée (par exemple par ajout de NaHCO₃). Pour l'azidation proprement dite, l'agent d'azidation (NaN₃) est additionné à une solution d'hydrazine à température ambiante ; le milieu réactionnel est alors agité pendant plusieurs heures à une température supérieure à ladite température ambiante. Ce mode d'obtention de ce composé nouveau est illustré dans la première partie de l'exemple 3 ;
- la 1-(2-aminoéthyl)-1-méthylhydrazine (ou 2-(1-méthylhydrazinyl)éthan-1-amine) : ce composé (de type 1-(2-aminoalkyl)-1-méthyl-hydrazine) est connu, commercialement disponible. Il peut être obtenu à partir du chlorhydrate de 2-chloroéthylamine, par condensation de la 2-chloroéthylamine sur un excès de monométhylhydrazine (MMH). La réaction est mise en oeuvre sous atmosphère inerte avec contrôle de son exothermicité ; et
- la 1-allyl-1-méthylhydrazine : ce composé est connu, il est commercialement disponible. Il peut être obtenu par allylation indirecte de la monométhylhydrazine (MMH). La réaction a été décrite par Smith et al. dans Synthetic Communications, 1990, 20 (2), 183-188). Sa mise en oeuvre est illustrée dans la première partie de l'exemple 5.

Ladite hydrazine précurseur, choisie parmi celles identifiées ci-dessus (toutes susceptibles d'être obtenues à partir de la monométhylhydrazine (MMH)), est alors oxydée, à des fins de dimérisation, pour conduire aux tétrazènes de formule **(Ia)** dans laquelle, respectivement, R1 = R2 = **-CH₂Cl** (i.e. au *(E)*-1,4-bis(2-chloroéthyl)-1,4-diméthyltétrazène), **-CH₂N₃** (i.e. au *(E)*-1,4-bis(2-azidoéthyl)-1,4-diméthyltétrazène), **-CH₂NH₂** (i.e. au *(E)*-1,4-bis(2-aminoéthyl)-1,4-diméthyltétrazène) ou **-CH=CH₂** (i.e. au *(E)*-1,4-diallyl-1,4-diméthyltétrazène). L'oxydation peut être mise en œuvre, comme indiqué ci-dessus, selon différents modes opératoires et tout particulièrement a) avec le diiode (I₂) en solution dans un solvant organique, notamment choisi parmi l'éthanol et l'éther diéthylique, et consistant avantageusement en l'éthanol ou b) avec KI₃ en solution aqueuse ou c) avec la monochloramine. Selon des variantes avantageuses :
- la 1-(2-chloroéthyl)-1-méthylhydrazine et la 1-(2-azidoéthyl)-1-méthylhydrazine sont oxydées avec la monochloramine. Une solution de monochloramine est opportunément préparée extemporanément ; et
- la 1-allyl-1-méthylhydrazine est oxydée avec le diiode en solution dans l'éther diéthylique. La solubilité des réactifs, 1-allyl-1-méthylhydrazine, d'une part, et diiode, d'autre part, est très satisfaisante dans ledit éther diéthylique.

Pour ce qui concerne le 1,4-diméthyltétrazène de formule **III** (le *(E)*-1,4-bis(2-hydroxyéthyl)-1,4-diméthyltétrazène), connu de l'art antérieur, il peut être alkylé, de façon conventionnelle, pour conduire aux di-éthers (symétriques) de formule **(Ia).** La réaction d'alkylation est schématisée ci-dessous pour l'obtention de l'éther d'allyle (di-éther (symétrique) de formule **(Ia)** dans laquelle R1 =R2 = **-CH₂O-CH₂-CH=CH₂).**

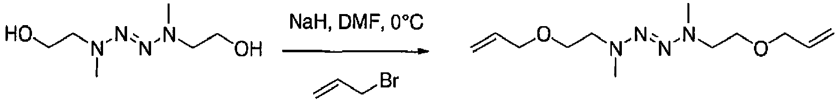

On préconise de procéder comme suit : utilisation du diméthylformamide (DMF) comme solvant, ajouts successifs d'hydrure de sodium en poudre (agent de déprotonation du diol) et du *(E)*-1,4-bis(2-hydroxyéthyl)-1,4-diméthyltétrazène (avec agitation) puis ajout, à basse température (0°C, par exemple), au goutte à goutte, de l'électrophile - bromure d'allyle, bromure de propargyle ou bromoacétonitrile (réactifs notamment commercialisés par la société Aldrich) ; agitation du milieu résultant à ladite basse température durant quelques heures (2h, par exemple) puis remontée à la température ambiante et maintien à ladite température ambiante durant plusieurs heures (18h, par exemple) ; élimination de l'hydrure de sodium qui n'aurait pas réagi par hydrolyse du milieu réactionnel (avec opportunément une solution saturée de NaCl), extraction du di-éther formé avec un solvant d'extraction, consistant avantageusement en l'éther diéthylique, et finalement élimination dudit solvant d'extraction utilisé par évaporation. Le di-éther obtenu est opportunément purifié par chromatographie sur gel de silice.

Pour ce qui concerne les autres 1,4-diméthyltétrazènes de formule **(Ia)**, à savoir :
- le *(E)*-1,4-bis(2-(1-méthylhydrazinyl)éthyl)-1,4-diméthyltétrazène (de formule **(Ia)** dans laquelle R1 = R2 = **-CH₂N(CH₃)(NH₂)),** et
- le *(E)*-1,4-bis(2-isocyanatoéthyl)-1,4-diméthyltétrazène (de formule **(Ia)** dans laquelle R1 = R2 = **-CH₂NCO),**

ils peuvent être obtenus, respectivement, à partir du *(E)*-1,4-bis(2-chloroéthyl)-1,4-diméthyltétrazène (de formule **(Ia)** dans laquelle R1 = R2 = **-CH₂Cl)** et du (E)-1,4-bis(2-aminoéthyl)-1,4-diméthyltétrazène (de formule **(Ia)** dans laquelle R1 = R2 = -**CH₂NH₂**). On peut parler de tétrazènes de formule **(Ia)** de seconde génération.

Pour la conversion des fonctions **-CH₂Cl** en fonctions **-CH₂N(CH₃)(NH₂),** on préconise une substitution nucléophile avec de la monométhylhydrazine (MMH). La réaction est mise en oeuvre sous atmosphère inerte, à froid (0°C, par exemple). Un mode opératoire de celle-ci est précisément décrit à l'exemple 4 ci-après. Pour la conversion des fonctions **-CH₂NH₂** en fonctions **-CH₂NCO,** on fait avantageusement intervenir du 1,1'-carbonyldiimidazole (pour une réaction "d'addition/élimination" ; le phosgène conventionnellement utilisé pour ce type de réaction pose des problèmes de compatibilité avec la fonction tétrazène), dans un solvant convenable, tel le tétrahydrofurane (THF), à une température supérieure à la température ambiante (50°C, par exemple).

Pour l'obtention des 1,4-diméthyltétrazènes, (di-)fonctionnalisés en position γ, de formule **(Ib),** on propose, dans le cadre du deuxième objet de l'invention, un procédé de préparation qui comprend :
- la mise à disposition d'une hydrazine précurseur choisie parmi la 1-(3-hydroxypropyl)-1-méthylhydrazine (ou 3-(1-méthylhydrazinyl)propan-1-ol), la 1-(3-aminopropyl)-1-méthylhydrazine (ou 3-(1-méthylhydrazinyl)propan-1-amine) et la 1-but-1-ènyl-1-méthylhydrazine, puis, par oxydation, l'obtention du dimère correspondant, tétrazène de formule (Ib) dans laquelle, respectivement, R3 = R4 = **-CH₂OH, -CH₂NH₂** ou **-CH=CH₂ ;**
- possiblement, la conversion du tétrazène de formule **(Ib)** dans laquelle R3 = R4 = **-CH₂NH₂,** avantageusement par réaction de ses fonctions amine (-NH₂) avec du 1,1'-carbonyldiimidazole, en le tétrazène de formule **(Ib)** dans laquelle R3 = R4 = **-CH₂NCO** ;
- possiblement, l'utilisation du tétrazène de formule **(Ib)** dans laquelle R3 = R4 = **-CH₂OH** :
   + pour obtenir, par alkylation de ses deux fonctions hydroxyle (-OH), un de ses diéthers symétriques de formule **(Ib)** (R3 = R4 = voir ci-dessus), ou
   + pour obtenir, par nitration, avec avantageusement le tétrafluoroborate de nitronium (NO₂BF₄), le tétrazène de formule **(Ib)** dans laquelle R3 = R4 = **-CH₂ONO₂,** ou
   + pour obtenir, par azidation, avantageusement en utilisant l'azoture de tosyle en présence d'une base faible telle le 1,8-diazabicyclo[5.4.0]undéc-7-ène (DBU), le tétrazène de formule **(Ib)** dans laquelle R3 = R4 = **-CH₂N₃** et le tétrazène de formule **(Ib)** dans laquelle R3 = **-CH₂OH** et R4 = **-CH₂N₃,** la fonction hydroxyle (-OH) de ce dernier pouvant être alkylée pour l'obtention des mono-éthers de formule **(Ib)** ; ou
   + pour obtenir, par action de l'azoture de diphénylphosphoryle, en présence d'une base faible telle le 1,8-diazabicyclo[5.4.0]undéc-7-ène (DBU) :
      - le *(E)*-1,4-bis(3-(diphénylphosphoryl)propyl)-1,4-diméthyltétrazène, intermédiaire convenant à l'obtention, selon une autre voie d'azidation, dudit tétrazène de formule **(Ib)** dans laquelle R3 = R4 = **-CH₂N₃,** ainsi qu'à l'obtention, par cyanation, du tétrazène de formule **(Ib)** dans laquelle R3 = R4 = **-CH₂CN** et à l'obtention, par substitution nucléophile avec de la monométhylhydrazine, du tétrazène de formule **(Ib)** dans laquelle R3 = R4 = **-CH₂N(CH₃)(NH₂),** ou
      - le *(E)*-1-(3-(diphénylphosphoryl)propyl)-4-(3-azidopropyl)-1,4-diméthyltétrazène, intermédiaire convenant à l'obtention, par cyanation, du tétrazène de formule **(Ib)** dans laquelle R3 = **-CH₂N₃** et R4 = **-CH₂CN** et à l'obtention, par substitution nucléophile avec de la monométhylhydrazine, du tétrazène de formule **(Ib)** dans laquelle R3 = **-CH₂N₃** et R4 = **-CH₂N(CH₃)(NH₂).**

Pour ce qui concerne la mise à disposition de l'hydrazine précurseur, on peut indiquer ce qui suit. L'hydrazine en cause peut consister en un produit connu, voire commercialisé. Il peut s'agir d'un produit nouveau. En tout état de cause, ladite hydrazine peut être obtenue à partir de la monométhylhydrazine (MMH). Ainsi :
- la 1-(3-hydroxypropyl)-1-méthylhydrazine : ce composé est connu. Il est commercialement disponible (voir ci-dessus), d'un coût très élevé. Il peut *a priori* être obtenu, en trois étapes, par la voie de synthèse décrite dans l'art antérieur pour le tétrazène de formule III. Il est avantageusement obtenu selon le mode de préparation préconisé présentement par les inventeurs (voir ci-dessus) : par condensation sous atmosphère inerte (argon, par exemple) d'un excès de monométhylhydrazine (MMH) sur le 3-chloropropanol. Ledit 3-chloropropanol est ajouté progressivement, avec contrôle de la température, sur ladite monométhylhydrazine (MMH). A l'issue de cet ajout, la réaction est poursuivie, avec contrôle de la température, avantageusement jusqu'à une conversion maximale, dudit 3-chloropropanol. Le chlorhydrate de méthylhydrazine formé conjointement est neutralisé par addition d'une base (avantageusement, NaOH). Le sel formé (NaCl, si NaOH est utilisé comme base) à l'issue de cette neutralisation est éliminé par filtration. Ce mode d'obtention, très avantageux, de ce composé connu est illustré dans la première partie de l'exemple 1' (les précisions données ci-dessus sur l'étape de condensation avec le 3-chloropropanol sont transposables à l'étape de condensation avec le 2-chloroéthanol pour l'obtention, selon la voie de synthèse préconisée présentement par les inventeurs, de la 1-(2-hydroxyéthyl)-1-méthylhydrazine (voir la première partie de l'exemple 1)) ;
- la 1-(3-aminopropyl)-1-méthylhydrazine : ce composé (de type 1-(3-aminoalkyl)-1-méthylhydrazine) est connu. Il peut être obtenu à partir du chlorhydrate de 3-chloropropylamine par condensation de la 3-chloropropylamine sur un excès de monométhylhydrazine (MMH). La réaction est mise en oeuvre sous atmosphère inerte avec contrôle de son exothermicité. Sa mise en oeuvre est illustrée dans la première partie de l'exemple 3' ci-après ; et
- la 1-but-1-ènyl-1-méthylhydrazine : ce composé est connu, il est commercialement disponible (d'un coût élevé). Il peut être obtenu par réaction de condensation entre la monométhylhydrazine (MMH) et le 4-bromobut-1-ène.

Ladite hydrazine précurseur, choisie parmi celles identifiées ci-dessus (toutes susceptibles d'être obtenues à partir de la monométhylhydrazine (MMH)), est alors oxydée, à des fins de dimérisation, pour conduire aux tétrazènes de formule **(Ib)** dans laquelle, respectivement, R3 = R4 = **-CH₂OH** (i.e. au *(E)*-1,4-bis(3-hydroxypropyl)-1,4-diméthyltétrazène (ou *(E)* 3,3'-(1,4-diméthyltétraaz-2-ène-1,4-diyl)bis(propan-1-ol)), - **CH₂NH₂** (i.e. au *(E)*-1,4-bis(3-aminopropyl)-1,4-diméthyltétrazène (ou *(E)*-3,3'-(1,4-diméthyltétraaz-2-éne-1,4-diyl)bis(propan-1-amine)) ou **-CH=CH₂** (i.e. au *(E)*-1,4-bis(but-1-ènyl)-1,4-diméthyltétrazène). L'oxydation peut être mise en oeuvre, comme indiqué ci-dessus, selon différents modes opératoires et tout particulièrement, comme pour l'obtention des tétrazènes « analogues » de formule **(Ia),** a) avec le diiode (I₂) en solution dans un solvant organique, notamment choisi parmi l'éthanol et l'éther diéthylique et consistant avantageusement en l'éthanol ou b) avec KI₃ en solution aqueuse ou c) avec la monochloramine. Selon des variantes avantageuses :
- la 1-(3-hydroxypropyl)-1-méthylhydrazine est oxydée avec KI₃ en solution aqueuse ou avec le diiode (I₂) en solution dans l'éthanol. Très avantageusement, elle est oxydée avec le diiode (I₂) en solution dans l'éthanol ; et
- la 1-but-1-ènyl-1-méthylhydrazine, tout comme la 1-allyl-1-méthylhydrazine, est oxydée avec le diiode (I₂) en solution dans l'éther diéthylique (solubilités très satisfaisantes).

A partir desdits dimères de formule **(Ib)** dans laquelle R3 = R4 = **-CH₂OH** ou **-CH₂NH₂,** tout particulièrement à partir de celui de formule **(Ib)** dans laquelle R3 = R4 = **-CH₂OH,** il est possible, comme indiqué ci-dessus, d'en préparer (directement (de seconde génération) ou indirectement) de nombreux autres.

Le tétrazène de formule **(Ib)** dans laquelle R3 = R4 = **-CH₂NCO** (i.e. le *(E)*-1,4-bis(2-isocyanatopropyl)-1,4-diméhyltétrazène) peut, tout comme son homologue de formule **(Ia)** dans laquelle R1= R2 = **-CH₂NCO,** être (directement) obtenu à partir de celui de formule **(Ib)** dans laquelle R3 = R4 = **-CH₂NH₂.** On fait avantageusement réagir, avec ledit tétrazène de formule **(Ib)** dans laquelle R3 = R4 = **-CH₂NH_{2,}** du 1,1'-carbonyldiimidazole. La réaction en cause est mise en oeuvre dans un solvant convenable, tel le tétrahydrofurane (THF), à une température supérieure à la température ambiante (50°C, par exemple).

On peut donc utiliser le tétrazène de formule **(Ib)** dans laquelle R3 = R4 = **-CH₂OH** pour préparer de nombreux autres tétrazènes de formule **(Ib),** et notamment :
- les di-éthers symétriques de formule **(Ib)** (éther d'allyle, de propargyle et d'acétonitrile) : ils sont directement obtenus, tout comme les di-éthers de formule **(Ia)** par des réactions d'alkylation entre ledit tétrazène de formule **(Ib)** dans laquelle R3 = R4 = **-CH₂OH** (= un diol) et un électrophile choisi parmi le bromure d'allyle, le bromure de propargyle et le bromoacétonitrile (voir ci-dessus) ;
- le tétrazène de formule **(Ib)** dans laquelle R3 = R4 = **-CH₂ONO₂** (i.e. le *(E)*-1,4-bis(3-nitrooxypropyl)-1,4-diméthyltétrazène) : les fonctions **-CH₂OH** du *(E)*1,4-bis(3-hydroxyprppyl)-1,4-diméthyltétrazène (tétrazène de formule (Ib) dans laquelle R3 = R4 = **-CH₂OH)** sont très avantageusement nitrées dans les conditions ci-après. Ledit tétrazène est mis en solution, à basse température (0°C, par exemple), dans un solvant, tel l'acétonitrile sec. Du tétrafluoroborate de nitronium (NO₂BF₄; notamment commercialement disponible auprès de la société Aldrich) est ajouté progressivement (avec contrôle de la température, à maintenir avantageusement en deçà de 5°C (au maximum à 5°C)) puis la réaction est laissée se poursuivre, sous agitation, à basse température. Ladite réaction est avantageusement stoppée par versement du milieu réactionnel dans de l'eau glacée. Le produit attendu (de seconde génération) peut être extrait au dichlorométhane et récupéré après évaporation dudit dichlorométhane ; et aussi
- pour obtenir, par azidation, avantageusement en utilisant l'azoture de tosyle (TsN₃) en présence d'une base faible telle le 1,8-DiazaBicyclo[5.4.0]Undéc-7-ène (DBU), les tétrazènes (de seconde génération) de formule **(Ib)** dans laquelle R3 = R4 = **-CH₂N₃** (i.e. le *(E)*-1,4-bis(3-azidopropyl)-1,4-diméthyltétrazène) et de formule **(Ib)** dans laquelle R3 **=-CH₂OH** et R4 = **-CH₂N₃** (i.e le (E)-1-(3-hydroxypropyl)-4-(3-azidopropyl)-1,4-diméthyltétrazène). Pour la mise en oeuvre de son azidation, le tétrazène de formule **(Ib)** dans laquelle R3 = R4 = **-CH₂OH** est dissous dans un solvant, tel le toluène. A l'issue de la mise en oeuvre de cette réaction d'azidation, les deux tétrazènes azidés - le mono-azidé et le di-azidé - sont obtenus en mélange. Ils peuvent être séparés par chromatographie en phase liquide sur gel de silice. L'obtention de ces deux tétrazènes en mélange ainsi que leur séparation sont illustrées à l'exemple 2'c ci-après. La fonction alcool du tétrazène de formule **(Ib)** dans laquelle R3 = **-CH₂OH** et R4 = **-CH₂N₃** peut être alkylée, de façon conventionnelle (voir les informations rappelées ci-dessus pour les doubles alkylations des fonctions hydroxyle (-OH) en position β et γ), pour conduire à un mono-éther de formule **(Ib).**

On peut, à partir du tétrazène de formule **(Ib)** dans laquelle R3 = R4 = **-CH₂OH,** selon une autre voie, obtenir ledit tétrazène de formule **(Ib)** dans laquelle R3 = R4 = **-CH₂N₃** ainsi que d'autres tétrazènes de formule **(Ib)** de l'invention. Cette autre voie comprend la préparation d'un intermédiaire phosphoré : le *(E)*-1,4-bis(3-(diphénylphosphoryl)propyl)-1,4-diméthyltétrazène. Conjointement audit *(E)*-1,4-bis(3-(diphénylphosphoryl)propyl)-1,4-diméthyltétrazène, on obtient le *(E)*-1-(3-(diphénylphosporyl)propyl)-4-(3-azidopropyl)-1,4-diméthyltétrazène qui, lui aussi, permet d'obtenir d'autres tétrazènes de formule (Ib).

Pour l'obtention des tétrazènes intermédiaires phosphorés, on active le tétrazène de formule **(Ib)** dans laquelle R3 = R4 = **-CH₂OH,** mis en solution, par exemple dans du toluène, avec un azoture phosphoré, l'azoture de diphénylphosphoryle (DPPA), en présence d'une base faible, telle le 1,8-DiazaBicyclo[5.4.0]Undéc-7-ène (DBU) (ces conditions d'azidation sont *per se* connues). On obtient un mélange des deux tétrazènes activés dans la mesure où l'une seulement des deux fonctions alcool ou les deux fonctions alcool ont été activées. Lesdits deux tétrazènes intermédiaires phosphorés peuvent être séparés par chromatographie en phase liquide sur gel de silice. Tout ceci est illustré à l'exemple 2'a ci-après.

Avec le tétrazène phosphoré di-activé, on peut, comme indiqué ci-dessus, préparer aussi le tétrazène de formule **(Ib)** dans laquelle R3 = R4 = **-CH₂N₃** (i.e. le *(E)*-1,4-bis(3-azidopropyl)-1,4-diméthyltétrazène). A cette fin d'azidation, ledit tétrazène phosphoré di-activé (opportunément dilué) peut être mis à réagir avec de l'azoture de sodium à chaud (à environ 50°C). Ceci est illustré à l'exemple 2'b.

Avec ledit tétrazène phosphoré di-activé , on peut aussi, par réaction avec NaCN ou KCN (par cyanation : substitution nucléophile du groupement phosphorylé par l'ion CN⁻), obtenir le tétrazène de formule **(Ib)** dans laquelle R3 = R4 = **-CH₂CN** (le (E)-1,4-bis(3-cyanopropyl)-1,4-diméthyltétrazène) ; on peut aussi, par substitution nucléophile avec la monométhylhydrazine (MMH), obtenir le tétrazène de formule **(Ib)** dans laquelle R3 = R4 = **-CH₂N(CH₃)(NH₂)** (i.e. le *(E)*-1,4-bis(3-(1-méthylhydrazinyl)-1,4-diméthyltétrazène). On comprend aisément qu'avec le tétrazène phosphoré mono-activé (le *(E)*-1-(3-(diphénylphosporyl)propyl)-4-(3-azidopropyl)-1,4-diméthyltétrazène), en mettant en oeuvre, respectivement, les mêmes réactions de cyanation et de substitution nucléophile avec la monométhylhydrazine (MMH), on obtient le tétrazène de formule **(Ib)** dans laquelle R3 = **-CH₂N₃** et R4 = **-CH₂CN** (i.e. le *(E)*-1-(3-cyanopropyl)-4-(3-azidopropyl)-1,4-diméthyltétrazène) et le tétrazène de formule **(Ib)** dans laquelle R3 = **-CH₂N₃** et R4 **= -CH₂N(CH₃)(NH₂)** (i.e. le *(E)*-1-(3-azidopropyl)-4-(3-(1-méthylhydrazinyl)propyl)-1,4-diméthyltétrazène).

Pour l'obtention des 1,4-diméthyltétrazènes β,γ-(di)fonctionnalisés de formule **(Ic)**, on propose un procédé de préparation qui comprend :
- la mise à disposition du 1-azido-3-(1-méthylhydrazinyl)propan-2-ol ou du 1-cyano-3-(1-méthylhydrazinyl)propan-2-ol (précurseur adéquat de type hydrazine), puis
- l'oxydation pour dimérisation, qui donne le tétrazène attendu, respectivement diazido (le *(E)*-1,4-bis(3-azido-2-hydroxypropyl)-1,4-diméthyltétrazène) ou dicyané (le *(E)*-1,4-bis(3-cyano-2-hydroxypropyl)-1,4-diméthyltétrazène).

Pour ce qui concerne la mise à disposition du précurseur adéquat de type hydrazine (de l'hydrazine précurseur), on peut indiquer ce qui suit. Lesdits précurseurs, à la connaissance des inventeurs, sont des composés nouveaux. Ils sont avantageusement obtenus comme suit :
- par ouverture de l'épichlorhydrine (ECH) en utilisant, respectivement, l'azoture de sodium ou le cyanure de potassium, puis
- condensation du produit résultant avec la monométhylhydrazine (MMH), pour, respectivement, l'obtention dudit 1-azido-3-(1-méthylhydrazinyl)propan-2-ol ou dudit 1-cyano-3-(1-méthylhydrazinyl)propan-2-ol.

L'ouverture de l'épichlorhydrine (ECH) par l'azoture de sodium conduit au 1-azido-3-chloropropan-2-ol. Elle a été décrite par Gharakanian et al., dans Biomacromolecules, 2015, 16 (6), 1802-1806. Sa mise en oeuvre est illustrée dans la première partie de l'exemple 1" ci-après. En faisant intervenir en lieu et place de l'azoture de sodium, du cyanure de potassium, on obtient le 1-cyano-3-chloropropan-2-ol.

La condensation dudit 1-azido-3-chloropropan-2-ol ou dudit 1-cyano-3-chloropropan-2-ol avec la MMH est avantageusement mise en oeuvre sous atmosphère inerte, par addition progressive dudit 1-azido-3-chloropropan-2-ol (voir la deuxième partie dudit exemple 1") ou dudit 1-cyano-3-chloropropan-2-ol sur la MMH.

L'oxydation de l'hydrazine précurseur ainsi obtenue (le 1-azido-3-(1-méthylhydrazinyl)-propan-2-ol ou le 1-cyano-3-(1-méthylhydrazinyl)propan-2-ol) peut être mise en oeuvre, comme indiqué ci-dessus, selon différents modes opératoires et tout particulièrement, comme pour l'obtention des tétrazènes « analogues » de formule **(Ia)** et **(Ib),** a) avec le diiode (I₂) en solution dans un solvant organique, notamment choisi parmi l'éthanol et l'éther diéthylique et consistant avantageusement en l'éthanol ou b) avec KI₃ en solution aqueuse ou c) avec la monochloramine. Selon une variante avantageuse, elle est mise en oeuvre avec KI₃ en solution aqueuse (voir la troisième partie dudit exemple 1").

On rappelle incidemment ici que, dans l'esprit général du procédé de l'invention - procédé qui comprend les deux étapes clés : mise à disposition d'une hydrazine précurseur convenable (avec utilisation de MMH) et oxydation de celle-ci pour dimérisation - les inventeurs ont également proposé une nouvelle voie de synthèse pour le tétrazène de formule **III** de l'art antérieur (voir ci-dessus ainsi que l'exemple 1 ci-après), nouvelle voie qui comprend la mise à disposition de la 1-(2-hydroxyéthyl)-1-méthylhydrazine (avantageusement par condensation (de MMH sur le 2-chloroéthanol)), puis par oxydation, la dimérisation de ladite hydrazine.

Selon un autre de ses objets, la présente invention concerne des composés intermédiaires, nouveaux, utiles à la préparation de tétrazènes de l'invention : 1,4-diméthyltétrazènes (di-)fonctionnalisés en position β, en position γ ou en positions β et γ. Elle concerne plus précisément :
- la 1-(2-azidoéthyl)-1-méthylhydrazine (utile à la préparation du 1,4-diméthyltétrazène β-fonctionnalisé de formule **(Ia)** dans laquelle R1 = R2 = **-CH₂N₃**),
- le *(E)*-1,4-bis(3-(diphénylphosphoryl)propyl)-1,4-diméthyltétrazène (utile à la préparation des 1,4-diméthyltétrazènes γ-fonctionnalisés de formule **(Ib)** dans laquelle R3 = R4 = **-CH₂N₃,** R3 = R4 = **-CH₂CN** et R3 = R4 = **-CH₂N(CH₃)(NH₂)),**
- le *(E)*-1-(3-(diphénylphosphoryl)propyl)-4-(3-azidopropyl)-1,4-diméthyltétrazène (utile à la préparation du 1,4-diméthyltétrazène γ-fonctionnalisé de formule **(Ib)** dans laquelle R3 = **-CH₂N₃** et R4 = **-CH₂CN** et à celle du 1,4-diméthyltétrazène γ-fonctionnalisé de formule (Ib) dans laquelle R3 = **-CH₂N₃** et R4 = **-CH₂N(CH₃)(NH₂)),**
- le 1-azido-3-(1-méthylhydrazinyl)propan-2-ol (utile à la préparation du 1,4-diméthyltétrazène β,γ-fonctionnalisé de formule **(Ic)** dans laquelle R5 = **-CH₂N₃**), et
- le 1-cyano-3-(1-méthylhydrazinyl)propan-2-ol (utile à la préparation du 1,4-diméthyltétrazène β,γ-fonctionnalisé de formule **(Ic)** dans laquelle R5 = **-CH₂CN).**

On se propose maintenant d'illustrer l'invention, de façon nullement limitative, par les figures et exemples ci-après.

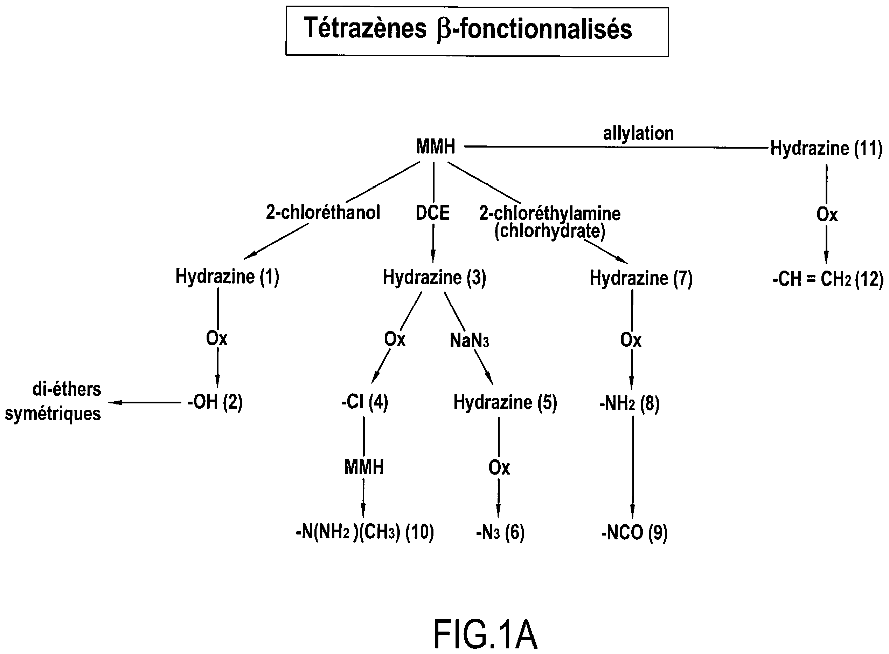

La figure 1A illustre, de façon schématique et nullement limitative, la préparation du tétrazène de formule III et des tétrazènes de formule **(Ia).**

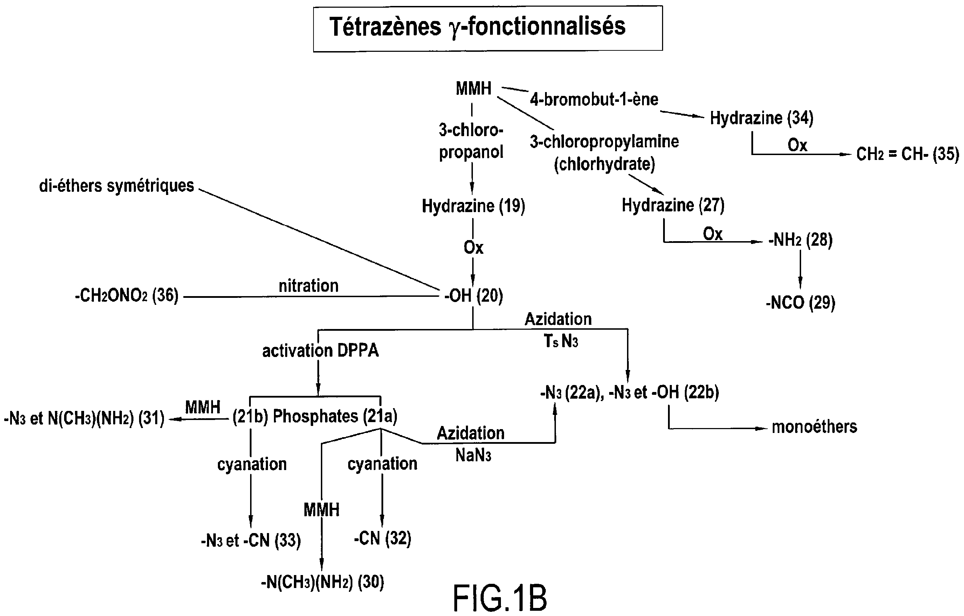

La figure 1B illustre, de façon schématique et nullement limitative, la préparation des tétrazènes de formule **(Ib)**.

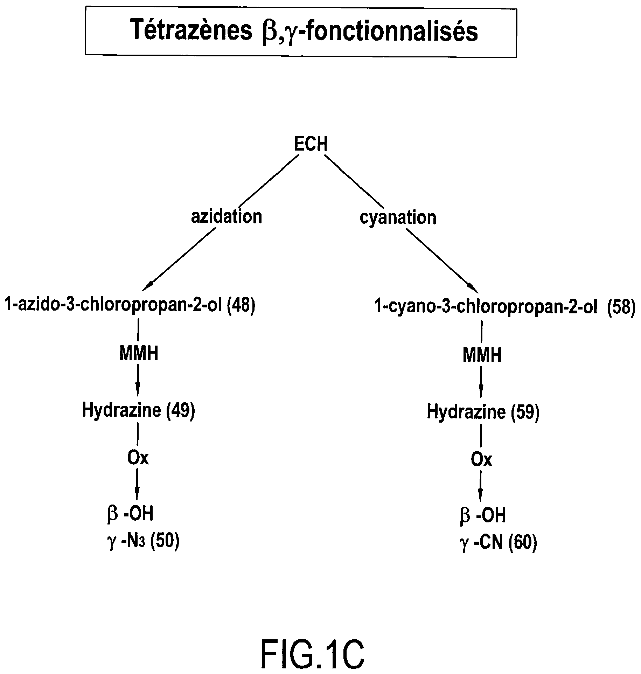

La figure 1C illustre, de façon schématique et nullement limitative, la préparation des tétrazènes de formule **(Ic)**.

Sur ladite figure 1A, on trouve, schématisée, la préparation des composés 1 à 6 et 10 à 12 des exemples 1 à 5 ci-après. On a, dans le même esprit, schématisé la préparation des di-éthers et des composés 7 (la 1-(2-aminoéthyl)-1-méthylhydrazine), 8 (le *(E)*-1,4-bis(2-aminoéthyl)-1,4-diméthyltétrazène) et 9 (le *(E)*-1,4-bis(2-isocyanatoéthyl)-1,4-diméthyltétrazène) (voir la description).

Sur ladite figure 1B, on trouve, schématisée, la préparation des composés 19, 20, 21a, 21b, 22a, 22b, 27, 28, 30 et 31 des exemples 1', 2'a, 2'b, 2'c, 3', 4' et 5' ci-après. On a, dans le même esprit, schématisé la préparation des di-éthers, des mono-éthers et des composés 29 (le *(E)*-1,4-bis(2-isocyanatopropyl)-1,4-diméhyltétrazène), 32 (le *(E)-1,4-*bis(3-cyanopropyl)-1,4-diméthyltétrazène), 33 (le *(E)*-1-(3-cyanopropyl)-4-(3-azidopropyl}-1,4-diméthyltétrazène), 34 (la 1-but-1-ènyl-1-méthylhydrazine ), 35 (le *(E)-*1,4-bis(but-1-ènyl)-1,4-diméthyltétrazène) et 36 (le (E)-1,4-bis(3-nitrooxypropyl)-1,4-diméthyltétrazène) (voir la description).

Sur ladite figure 1C, on trouve, schématisée, la préparation des composés 48-50 de l'exemple 1" ci-après. On a, dans le même esprit, schématisé la préparation des composés 58 (le 1-cyano-3-chloropropan-2-ol), 59 (le 1-cyano-3-(1-méthylhydrazinyl)propan-2-ol) et 60 (le *(E)*-1,4-bis(3-cyano-2-hydroxypropyl)-1,4-diméthyltétrazène) (voir la description).

### Exemple 1 : procédé de synthèse (nouveau) du (E)-1,4-bis(2-hydroxyéthyl)-1,4-diméthyltétrazène (2 = tétrazène de formule III de l'art antérieur)

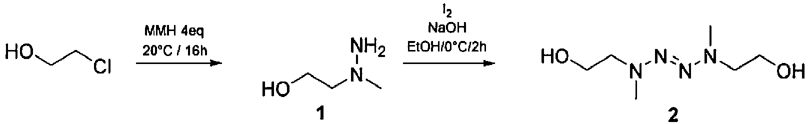

### Synthèse de la 1-(2-hydroxyéthyl)-1-méthylhydrazine (1)

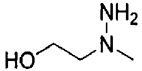

Trois cycles de vide/argon ont été effectués dans un ballon double enveloppe de 250 mL afin d'y installer une atmosphère inerte. La monométhylhydrazine (50 mL, 950 mmol) a été ajoutée et le milieu a été maintenu à 20°C au moyen d'un cryostat. Une addition lente (6 h) de 2-chloroéthanol (16 mL, 240 mmol) a alors été mise en place et la réaction s'est poursuivie pendant la nuit. Le lendemain matin, le milieu a été refroidi à 0°C et une solution aqueuse saturée de NaOH (25 mL) a été additionnée lentement afin de neutraliser le chlorhydrate de méthylhydrazine formé au cours de la réaction. Le milieu a ensuite été évaporé au maximum sous pression réduite puis dilué dans l'acétonitrile (50 mL), ce qui a permis de faire précipiter les sels qui ont ensuite été filtrés. Cette opération de purification par précipitation a été répétée 3 fois pour obtenir un produit pur sous la forme d'un liquide visqueux (9,2 g, rendement 43%).

RMN ¹H (D₂O, 25°C, 300MHz) : δ (ppm) = 2,47 (s, 3H, NMe), δ (ppm) = 2,71 (t, 3JHH = 6 Hz, 2H, NCH₂), δ (ppm) = 3,72 (t, 3JHH = 6 Hz, 2H, CH₂OH).

On rappelle à toutes fins utiles que ce produit est commercialement disponible.

### Synthèse du (E)-1,4-bis(2-hydroxyéthyl)-1,4-diméthyltétrazène (2)

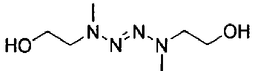

La 1-(2-hydroxyéthyl)-1-méthylhydrazine (1,05 g, 11,7 mmol) a été introduite dans un ballon de 100 mL puis diluée dans 45 mL d'éthanol (C=0,25 M). De l'hydroxyde de sodium a ensuite été ajouté (940 mg, 23,5 mmol) puis le milieu réactionnel a été refroidi à 0°C au moyen d'un bain de glace. Le diiode (2,97 g, 11,7 mmol) a alors été additionné par portion sous forme de billes, à une température comprise entre 0 et 3°C. Le milieu réactionnel a ensuite été agité pendant 2 h à 0°C au terme de l'ajout. Le solvant a été évaporé et le résidu a été purifié sur colonne de silice avec un éluant dichlorométhane/méthanol (gradient 0 %/5 % MeOH sur 30 minutes). On a obtenu un liquide visqueux de couleur jaune (561 mg, rendement 53%).
RMN ¹H (300 MHz, CDCl₃) : δ (ppm) = 2,82 (s, 6H, CH₃) ; 3,32 (t, 4H, J = 6 Hz, CH₂N) ; 3,84 (t, 4H, J = 6 Hz, CH₂O)
RMN ¹³C (100 MHz, CDCl₃): δ (ppm) = 38,5 (CH₃) ; 57,6 (CH₂N) ; 60,6 (CH₂O)
IR (golden gâte, rel. int.): v (cm⁻¹) = 586(m) ; 1002(s) ; 1261(m) ; 1357(m) ; 1465(m) ; 1652(w) ; 2851(m) ; 3326(s)
MS (ESI Tof) : m/z = 199,1 [M+Na]⁺

### Tétrazènes β-fonctionnalisés de formule (Ia)

### Exemple 2

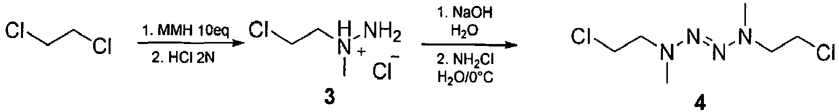

### Synthèse du chlorhydrate de 1-(2-chloroéthyl)-1-méthylhydrazine (3) (d'après H.Böhme et al. dans Arch. Pharmaz. 307/74, 272-277).

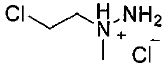

Trois cycles de vide/argon ont été effectués dans un réacteur double enveloppe de 1 L afin d'y installer une atmosphère inerte. Le 1,2-dichloroéthane (500 mL, 6.23 mol) a été ajouté et le milieu a été maintenu à 20°C au moyen d'un cryostat. Une addition lente (6 h) de méthylhydrazine (32 mL, 650 mmol) a alors été mise en place et le mélange réactionnel a été maintenu sous agitation à 20°C pendant 3 jours. Le milieu réactionnel a ensuite été filtré pour éliminer la majeure partie du chlorhydrate de monométhylhydrazine co-produit. Il a ensuite été traité en utilisant une solution de HCl 0,2N, ce qui a permis, dans un premier temps, d'éliminer le chlorhydrate de MMH, puis d'extraire ensuite l'hydrazine chlorée sous forme de chlorhydrate. Les phases aqueuses contenant exclusivement de la 1-(2-chloroéthyl)-1-méthylhydrazine ont été rassemblées et l'eau a été évaporée sous pression réduite jusqu'à l'obtention d'un solide jaune (16,38 g ; rendement 35%).
Aspect : solide jaune

1H NMR (300 MHz, D2O) : δ (ppm) = 3,03 (s, 3H, NMe), δ (ppm) = 3,56 (t, 3JHH = 6 Hz, 2H, NCH₂), δ (ppm) = 3,91 (t, 3JHH = 6 Hz, 2H, CH₂Cl).

13C NMR (D₂O, 300 MHz, 25°C) δ (ppm) = 38,09 (NMe), δ (ppm) = 45,15 (CH₂Cl), δ (ppm) = 60,09 (NCH₂).

### Synthèse du (E)-1,4-bis(2-chloroéthyl)-1,4-diméthyltétrazène (4)

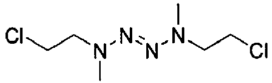

**Préparation d'une solution aqueuse de monochloramine 1M:** 10 mL d'une solution aqueuse alcalinisée d'hypochlorite de sodium ([OCl⁻] = 2,20 mol.L⁻¹ ; [NaOH] = 0,45 mol.L⁻¹) ont été additionnés goutte à goutte à 10 mL d'une solution aqueuse de chlorure d'ammonium (2,42 mol.L⁻¹) à une température comprise entre -7° et -5°C. A l'issue de la réaction instantanée, la solution de monochloramine obtenue titre 1,08 mol.L⁻¹ (dosage par iodométrie). Cette solution a dû être utilisée rapidement (puisqu'elle ne pouvait être conservée à 0°C que pendant 30 min).

Dans un ballon de 100 mL, on a introduit 2,04 g (13,8 mmol) de chlorhydrate de 1-(2-chloroéthyl)-1-méthylhydrazine et 10 mL d'eau. Cette solution a été refroidie à 0°C, puis on a ajouté une solution d'hydroxyde de sodium à 1 mol.L⁻¹ jusqu'à obtenir un pH de 9 dans le milieu réactionnel. La solution de monochloramine a ensuite été ajoutée goutte à goutte à 0°C (6 mL, 1,08 mol.L⁻¹), puis le mélange réactionnel a été agité pendant 30 min à 0°C. Il a ensuite été extrait avec de l'éther diéthylique (3×20 mL). Les phases éthérées ont été rassemblées, lavées avec une solution saturée de NaCl, séchées sur Na₂SO₄ et évaporées. Le brut a été purifié sur colonne de silice (Cyclohexane/Acétate d'éthyle 8:2). Le (E)-1,4-bis(2-chloroéthyl)-1,4-diméthyltetrazène a été obtenu sous forme d'un liquide jaune (rendement 43%).
Aspect : liquide jaune
RMN ¹H (400 MHz, CDCl₃) : δ (ppm) = 2,87 (s, 6H, CH₃) ; 3,54 (t, 4H, J = 7,0 Hz, CH₂N) ; 3,67 (t, 4H, J = 7,0 Hz, ClCH₂)
RMN ¹³C (100 MHz, CDCl₃) δ (ppm) = 38,4 (CH₃) ; 41,4 (ClCH₂) ; 56,7 (CH₂N)
RMN ¹⁵N (50 MHz, CDCl₃) δ (ppm) = 126,0 (N-N=N) ; 394,7 (N=N)
UV (Et₂O) : λₘₐₓ = 280 nm ; épaulement : 250 nm
IR (golden gate, rel. int.) : v (cm⁻¹) = 594(s) ; 661(m) ; 737(m) ; 1003(s) ; 1099(m) ; 1255(m) ; 1466(m) ; 2961(m)
DSC (-50 à 250°C ; 5°C/min) : T_{c} (pic onset) = -42,56°C (exo), -119,0 J.g⁻¹ ; T_{f} (pic onset) = -14,47°C (endo), 89,0 J.g⁻¹ ; T_{d} (pic onset) = 107,63 °C (exo), -1624,2 J.g⁻¹
GC/MS : m/z = 212

### Exemple 3

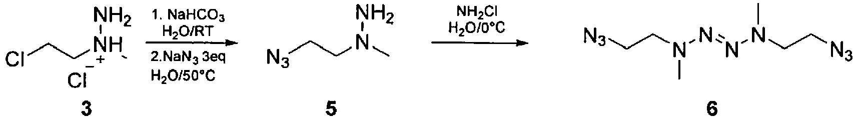

### Synthèse de la 1-(2-azidoéthyl)-1-méthylhydrazine (5)

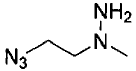

Dans un ballon bicol de 250 mL, on a introduit le chlorhydrate de 1-(2-chloroéthyl)-1-méthylhydrazine (7,35 g ; 50,9 mmol) dans 100 mL d'eau. La solution aqueuse a été refroidie à 0°C et neutralisée par ajout de NaHCO₃ (4,28 g ; 51 mmol). Après retour à température ambiante, 10,17 g de NaN₃ (156 mmol) ont été additionnés par portion dans le mélange réactionnel qui a ensuite été agité pendant 6 heures à 50°C. A la fin de la réaction, la solution a été basifiée avec de l'hydroxyde de potassium (30 g) et extraite en continu avec de l'éther diéthylique (150 mL) pendant 10 h. La phase éthérée a été séchée sur Na₂SO₄ et évaporée. La 1-(2-azidoéthyl)-1-méthylhydrazine a été obtenue sous forme d'un liquide jaune (rendement 24%).
Aspect : liquide jaune
RMN ¹H (400 MHz, CDCl₃) δ (ppm) = 2,54 (s, 3H, CH₃) ; 2,59 (t, 2H, J = 5,7 Hz, CH₂N) ; 2,89 (s, 2H, NH₂) ; 3,47 (t, 2H, J = 5,7 Hz, N₃CH₂)
RMN ¹³C (100 MHz, CDCl₃) δ (ppm) = 48,8 (CH₃) ; 51,1 (N₃CH₂) ; 60,4 (CH₂N)
RMN ¹⁵N (50 MHz, CDCl₃) δ (ppm) = 64,5 (N(CH₃)NH₂) ; 69,5 (N=N=N-CH₂) ; 92,4 (NH₂) ; 209,6 (CH₂-N=N=N) ; 248,4 (N=N=N)
UV (Et₂O) : λₘₐₓ₁ = 244 nm, λₘₐₓ₂ = 302 nm
IR (golden gate, rel. int.) : ν (cm⁻¹) = 1066(w) ; 1278(m) ; 1446(m) ; 1599(w) ; 2094(s) ; 2945(w)
HRMS (ESI Tof) [M+H]⁺ : m/z = 116,0931 (théorique), 116,0932 (mesuré), 0,9 ppm ; [2M+Na]⁺: m/z = 253,1638
DSC (-50 à 250°C ; 5°C/min) : T_{d} (pic onset) = 152,9°C (exo) -275,1 J.g⁻¹

### Synthèse du (E)-1,4-bis(2-azidoéthyl)-1,4-diméthyltétrazène (6)

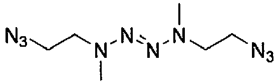

A une solution de 1-azidoéthyl-1'-méthylhydrazine (1,22 g ; 10 mmol) dans l'eau (10 mL) à 0°C, ont été ajoutés 50 mL d'une solution aqueuse de monochloramine (96 mmol.L⁻¹ ; 4,8 mmol) obtenue selon le protocole décrit dans l'exemple 2. Le mélange réactionnel a été agité pendant 1 heure à 0°C puis extrait à l'éther diéthylique. Les phases organiques réunies ont été séchées sur Na₂SO₄ et concentrées par évaporation du solvant. Un liquide jaune a été obtenu, contenant le tétrazène attendu et l'excès d'hydrazine. Une purification sur colonne de silice a permis de purifier le composé en utilisant le dichlorométhane comme éluant (Rf = 0,5). Le (E)-1,4-bis(2-azidoethyl)-1,4-diméthyltetrazène a été obtenu avec un rendement de 72% (390 mg) sous la forme d'un liquide jaune pâle.
RMN ¹H (400 MHz, CDCl₃) : δ (ppm) = 2,86 (s, 6H, CH₃) ; 3.41 (t, 4H, J = 6,4 Hz, CH₂N) ; 3,48 (t, 4H, J = 6,4 Hz, N₃CH₂)
RMN ¹³C (100 MHz, CDCl₃): δ (ppm) = 37,9 (CH₃) ; 48,8 (N₃CH₂) ; 54,0 (CH₂N)
RMN ¹⁵N (CDCl₃) (ppm): δ = 123,5 (**N**-N=N) ; 210,5 (CH₂-N=N=**N**) ; 248,2 (N=**N**=N) ; 394,7 (N-**N**=N)
UV (Et₂O) : λₘₐₓ = 281 nm
IR (golden gate, rel. int.): v (cm⁻¹) = 585 (m) ; 1013 (s) ; 1272 (s) ; 1466 (m) ; 2088 (s) ; 2859 (w)
HRMS (ESI Tof) [M+H]⁺: m/z = 227,1476 (théorique), 227,1473 (mesuré), 1,3 ppm DSC (-50 à 250°C ; 5°C/min) : T_{f} (pic onset) = -13,2°C (endo), 95,5 J.g⁻¹ ; Td (pic onset) = 137,6°C (exo), -372,9 J.g⁻¹

### Exemple 4

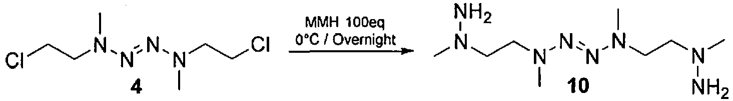

### Synthèse du (E)1,4-bis(2-(1-méthylhydrazinyl)éthyl)-diméthyl-1,4-diméthyltétrazène (10)

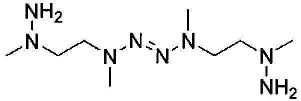

Trois cycles de vide/argon ont été effectués dans un schlenk afin d'y installer une atmosphère inerte. Le (E)-1,4-bis(2-chloroéthyl)-1,4-diméthyltetrazène (761 mg, 3.57 mmol) a été introduit dans le schlenk et refroidi à 0°C. La monométhylhydrazine (15 ml, 285 mmol) a ensuite été ajoutée et la solution a été agitée pendant 24 h à 0°C. 30 ml d'une solution saturée de NaHCO₃ ont ensuite été ajoutés puis le milieu réactionnel a été extrait au dichlorométhane. Les phases organiques ont été séchées sur MgSO₄ et concentrées sous vide, ce qui a permis d'obtenir une huile jaune (624 mg, rendement 75%).
RMN ¹H (400 MHz, CDCl₃) : δ (ppm) = 2,51 (s, 6H, CH₃NNH₂) ; 2,68 (t, 4H, CH₂NNH2) ; 2,81 (s, 6H, N₄CH₃) ; 3,39 (t, 4H, N₄CH₂)
RMN ¹³C (100 MHz, CDCl₃) : δ (ppm) = 37,9 (CH₃NNH₂) ; 49,9 (N₄CH₃) ; 52,9 (CH₂NNH₂) ; 59,7 (N₄CH₂)
HRMS (ESI-Tof) [M+H]⁺ : m/z = 233,2197 (théorique), 233,2187 (mesuré), 4,2 ppm

### Exemple 5

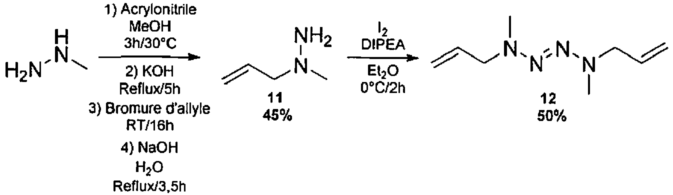

### Synthèse de la 1-allyl-1-méthylhydrazine (11) (d'après Smith et al., dans Synthetic Communications, 1990, 20 (2), 183-188)

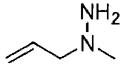

Une atmosphère inerte a été instaurée dans un ballon de Schlenk de 100 mL. La méthylhydrazine (2,63 mL, 50 mmol) a été ajoutée et diluée dans 25 mL de méthanol. Le milieu a été thermostaté à 30°C au moyen d'un cryostat puis l'acrylonitrile (3,60 mL, 55 mmol) a été additionné sur 2,5 h. A la fin de l'ajout le milieu a été agité durant 30 minutes supplémentaires à 30°C puis des pastilles de KOH (4,5 g, 89 mmol) ont été ajoutées et le milieu a été porté à reflux durant 5 h. Le milieu a ensuite été laissé à refroidir à température ambiante. Le bromure d'allyle (4,32 mL, 49,99 mmol) a alors été ajouté par portions. La réaction s'est poursuivie à température ambiante durant la nuit. Le lendemain matin le solvant a été évaporé sous pression réduite puis le résidu a été dilué dans une solution aqueuse de NaOH (6 N, 75 mL). Ensuite, le milieu a été porté à reflux durant 3,5 h. Une distillation sous pression réduite a permis d'obtenir un mélange d'eau et du produit attendu. Un test à l'acide phosphomolybdique a permis de déterminer la présence ou non du produit souhaité dans les fractions distillées. Le produit pur a été obtenu par démixtion sur pastilles de KOH sous la forme d'un liquide jaune (1,95 g, rendement 45%).

RMN ¹H (CDCl₃, 25°C, 300MHz) : δ =2,46 ppm (s, 3H, NCH3), δ = 3,10 ppm (dt, 2H, NC***H₂***, J₁=6,57Hz, J₂=1,21Hz), δ =5,18 ppm (t, 1H, H Allylique terminal Cis, J=1,14Hz), δ =5,87 ppm (m, 1H, NCH₂C***H***CH₂), δ =5,22 ppm (m, 1H, H Allylique terminal Trans). RMN ¹³C (CDCl₃, 25°C, 300MHz) : δ =66,71 ppm (N***C***H₂CHCH₂), δ = 48,80 ppm (N***C***H₃), δ = 118,97 ppm (NCH₂CH***C***H₂), δ =134,58 ppm (NCH₂***C***HCH₂).

### Synthèse du (E)-1,4-diallyl-1,4-diméthyltétrazène (12)

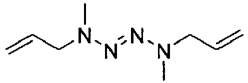

L'hydrazine **11** (202,1 mg, 2,35 mmol) a été introduite dans un ballon monocol de 25 mL et diluée dans de l'éther diéthylique (9 mL, C=0,25 M). La N,N-diisopropyléthylamine (DIPEA) (0,850 mL, 4,92 mmol) a été ajoutée et le milieu a été refroidi à 0°C au moyen d'un bain de glace. Le diiode (596,1 mg, 2,35 mmol) a alors été ajouté en billes par portions puis la réaction s'est poursuivie 2 h à 0°C. Le précipité blanc de DIPEA.HI a été filtré et le solvant a été évaporé sous pression réduite sans chauffer afin d'éviter l'évaporation du produit. Le résidu a été dilué dans 3 mL de dichlorométhane puis purifié sur colonne de silice avec un éluant dichlorométhane pur. Le produit a été obtenu sous la forme d'un liquide jaune (98,50 mg, rendement 50%).

RMN ¹H (CDCl₃, 25°C, 300 MHz) : δ =2,73 ppm (s, 6H, NCH3), δ =3,84 ppm (d, 4H, NC***H₂***), δ = 5,14-5,23 ppm (m, 4H, H Allyliques terminaux), δ =5,87 ppm (m, 2H, NCH₂C***H***CH₂).

RMN ¹³C (CDCl₃, 25°C, 300 MHz) : δ = 58,15 ppm (N***C***H₂CHCH₂), δ = 37,01 ppm (N***C***H₃), δ = 117,78 ppm (NCH₂CH***C***H₂), δ = 134,47 ppm (NCH₂***C***HCH₂).

HRMS (ESI⁺) : [M+H]⁺ m/z = 169,1448 (théorique); 169,1444 (mesurée); 2,0 ppm. [M+Na]⁺ m/z = 191,1267 (théorique); 191,1264 (mesurée); 1,4 ppm.

IR (Golden Gate, rel. int.) : v (cm⁻¹) = 3078, 2844, 1643, 1462, 1418, 1332, 1253, 1167, 1084, 1009, 991, 917.

DSC (25°C à 350°C ; 2°C/min) : T_{d} (pic onset) = 133°C (-1290J/g).

UV/Vis : λₘₐₓ = 280 nm.

### Tétrazènes γ-fonctionnalisés de formule (Ib)

### Exemple 1'

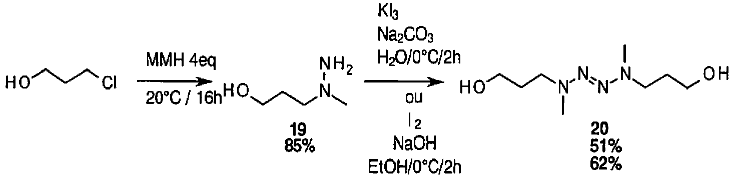

### Synthèse de la 1-(3-hydroxypropyl)-1-méthylhydrazine (19)

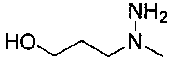

Trois cycles de vide/argon ont été effectués dans un ballon double enveloppe de 250 mL afin d'y installer une atmosphère inerte. La méthylhydrazine (50 mL, 950 mmol) a été ajoutée et le milieu a été maintenu à 20°C au moyen d'un cryostat. Une addition lente (6 h) de 3-chloropropan-1-ol (20 mL, 239 mmol) a alors été mise en place et la réaction s'est poursuivie pendant la nuit. Le lendemain matin, le milieu a été refroidi à 0°C et une solution aqueuse saturée de NaOH (25 mL) a été additionnée lentement afin de neutraliser le chlorhydrate de méthylhydrazine formé au cours de la réaction. Le milieu a ensuite été évaporé au maximum sous pression réduite puis dilué avec 25 mL de dichlorométhane. Après filtration le solvant a été évaporé puis les sels résiduels ont été précipités dans l'acétonitrile (50 mL). Le milieu a été filtré et le solvant a été évaporé. Cette opération de purification par précipitation a été répétée 3 fois pour obtenir un produit pur sous la forme d'un liquide visqueux (21,06 g, rendement 85%).

RMN ¹H (CDCl₃, 25°C, 300 MHz) : δ = 1,74 ppm (quint, 2H, CH₂C***H₂***CH₂OH), δ = 3,52 ppm (s(b), 3H, CH₂O***H*** et NN***H₂***), δ = 2,47 ppm (s, 3H, NC***H₃***), δ = 2,61 ppm (t, 2H, NC***H₂***CH₂CH₂OH), δ = 3,70 ppm (t, 2H, NCH₂CH₂C***H₂***OH).

RMN ¹³C (CDCl₃, 25°C, 300 MHz) : δ = 29,63 ppm (NCH₂***C***H₂CH₂OH), δ = 63,20 ppm (N***C***H₂CH₂CH₂OH), δ = 62,59 ppm (NCH₂CH₂***C***H₂OH), δ = 50,42 ppm (N***C***H₃).

On rappelle à toutes fins utiles que ce produit est commercialement disponible.

### Synthèse du (E)-1,4-bis(3-hydroxypropyl)-1,4-diméthyltétrazène (20)

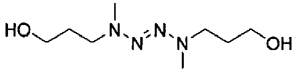

### Méthode 1 : Utilisation de l'eau comme solvant

L'hydrazine **19** (2,53 g, 24,24 mmol) a été introduite dans un ballon monocol de 250 mL puis diluée dans 78 mL (C=0,25 M) d'eau distillée. Deux équivalents de Na₂CO₃ (5,4 g, 50,9 mmol) ont été ajoutés et le milieu a été refroidi à 0°C à l'aide d'un bain de glace. Lorsque le milieu a atteint une température de 0°C, une solution aqueuse de KI₃ d'une concentration de 1,28 M (19 mL, 24,32 mmol) a été additionnée au goutte à goutte et la réaction s'est poursuivie durant 2 h à 0°C. Le reste de KI₃ n'ayant pas réagi a alors été neutralisé en additionnant du thiosulfate de sodium (1 g, 6,3 mmol) et en laissant agiter le milieu durant 5 minutes. Le solvant a ensuite été co-évaporé avec de l'éthanol afin d'obtenir un solide brun. Celui-ci a été lavé avec 3 portions de 50 mL de dichlorométhane puis les phases organiques ont été regroupées et évaporées. Le tétrazène **20** est obtenu par purification sur colonne de silice avec un éluant dichlorométhane/méthanol (gradient 0%/5% MeOH sur 30 minutes). On a obtenu un liquide visqueux de couleur jaune (1,294 g, rendement 51%).

### Méthode 2 : Utilisation de l'éthanol comme solvant

L'hydrazine **19** (5,00 g, 48,01 mmol) a été introduite dans un ballon monocol de 500 mL puis diluée dans l'éthanol (190 mL, C=0,25 M). De l'hydroxyde de sodium sous forme poudreuse a ensuite été ajouté (4,06 g, 101,51 mmol) puis le milieu réactionnel a été refroidi à 0°C au moyen d'un bain de glace. Le diiode (12,21 g, 48,11 mmol) a alors été additionné par portion sous forme de billes. On a observé une exothermicité modérée, l'ajout a donc été dosé afin de ne pas réchauffer le milieu au-delà de 3°C. La réaction s'est poursuivie durant 2 h à 0°C au terme de l'ajout. Le solvant a été évaporé sous pression réduite et le résidu a été dilué dans le dichlorométhane (10mL). Après filtration, le filtrat a été récupéré et purifié sur colonne de silice avec un éluant dichlorométhane/méthanol (gradient 0 %/5 % MeOH sur 30 minutes). On a obtenu un liquide visqueux de couleur jaune (3,04 g, rendement 62%).

RMN ¹H (CDCl₃, 25°C, 300 MHz) : δ = 1,84 ppm (quint, 4H, CH₂C***H₂***CH₂OH), δ = 2,42 ppm (s(b), 2H, CH₂O***H***), δ = 2,80 ppm (s, 6H, NC***H₃***), δ = 3,34 ppm (t, 4H, NC***H₂***CH₂CH₂OH), δ = 3,70 ppm (t, 4H, NCH₂CH₂C***H₂***OH).

RMN ¹³C (CDCl₃, 25°C, 300 MHz) : δ = 30,06 ppm (NCH₂***C***H₂CH₂OH), δ = 52,73 ppm (N***C***H₂CH₂CH₂OH), δ = 61.29 ppm (NCH₂CH₂***C***H₂OH), δ = 38,42 ppm (N***C***H₃).

HRMS (ESI⁺) : [M+Na]⁺ m/z = 227,1482 (théorique); 227,1478 (mesurée); 1,7 ppm. [2M+Na]⁺ m/z = 431,3079 (théorique); 431,3065 (mesurée); 3,2 ppm.

IR (Golden Gate, rel. int.) : v (cm⁻¹) = 3313, 2932, 2858, 1466, 1040, 994.

DSC (25°C à 350°C ; 2°C/min) : T_{d} (pic onset) = 120°C (-590J/g).

UV : λₘₐₓ = 280 nm.

### Exemple 2'a

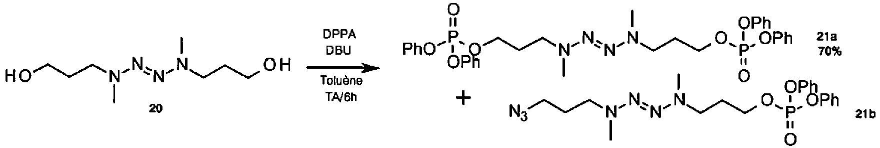

### Synthèse du (E)-1,4-bis(diphénylphosphoryl)propyl)-1,4-diméthyltétrazène (21a) et du (E)-1-(3-(diphénylphosphoryl)-4-(3-azidopropyl)-1,4-diméthyltétrazène (21b)

A toutes fins utiles, on reproduit ci-après la formule de l'intermédiaire **21a** :

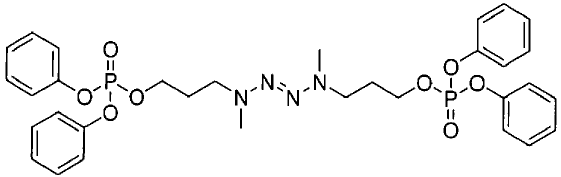

Le tétrazène **20** (1004,8 mg, 4,92 mmol) a été introduit dans un ballon monocol de 10 mL et dilué dans 10 mL de toluène. L'azoture de diphénylphosphoryle (DPPA) (2,11 mL, 9,80 mmol) a été ajouté puis le DBU (1,8-DiazaBicyclo[5.4.0]Undéc-7-ène) (1,46 mL, 9,80 mmol) a été additionné au goutte à goutte et la réaction s'est poursuivie durant la nuit à température ambiante. Le toluène a ensuite été évaporé et le brut réactionnel a été dilué dans 3 mL de dichlorométhane. Une purification sur colonne de silice avec un éluant cyclohexane/acétate d'éthyle (gradient 20-50% AcOEt sur 30 minutes) a permis d'isoler les produits **21a et 21b** : le produit **21a** sous la forme d'un liquide visqueux jaune pâle (1,225 g, 38 %), le produit **21b** sous la forme d'un liquide jaune (703,5 mg, 31%) (avec un temps de rétention plus court que le produit **21a**).

### Caractérisation 21a

RMN ¹H (CDCl₃, 25°C, 300 MHz) : δ =2,01 ppm (quint, 4H, CH₂C***H*₂**CH₂OP, J=6,66Hz), δ =2,70 ppm (s, 6H, NC***H₃***), δ = 3,23 ppm (t, 4H, NC***H₂***CH₂CH₂OP, J=6,87Hz), δ = 4,34 ppm (quad, 4H, NCH₂CH₂C***H₂***OP, J = 7,47Hz), δ = 7,16-7,36 ppm (m, 20H, Aromatiques).

RMN ¹³C (CDCl₃, 25°C, 300 MHz) : δ = 28,30 ppm (d, NCH₂***C***H₂CH₂OP, J = 27,42Hz), δ = 38,11 ppm (N***C***H₃), δ = 51,60 ppm (N***C***H₂CH₂CH₂OH), δ = 67,49 ppm (d, NCH₂CH₂***C***H₂OP, J = 25,38 Hz), δ = 120,17 ppm (d, POC(***C***H)₂(CH)₂C, J = 19,53Hz), δ = 125,47 ppm (POC(CH)₂(CH)₂***C***), δ = 129,92 ppm (POC(CH)₂(***C***H)₂C), δ = 150,68 ppm (d, PO***C***(CH)₂(CH)₂C, J = 28,65 Hz).

RMN ³¹P {¹H} (CDCl₃, 25°C, 300 MHz) : d= -11,91 ppm (CH₂O***P***(OPh)₂).

HRMS (ESI⁺) : [M+H]⁺ m/z = 669,2219 (théorique); 669,2238 (mesuré.); 2,8 ppm. [M+Na]⁺ m/z = 691,2058 (théorique); 691,2057 (mesurée); 0,1 ppm.

IR (Golden Gate, rel. int.) : v (cm⁻¹) = 3069, 2964, 2850, 1589, 1487, 1456, 1285, 1187, 936.

DSC (25°C à 350°C ; 2°C/min) : T_{d} (pic onset) = 78°C (-456J/g).

UV : λₘₐₓ = 280 nm

### Caractérisation 21b

RMN ¹H (CDCl₃, 25°C, 300 MHz) : δ = 1,88 ppm (quint, 2H, CH₂CH₂CH₂N₃, J=6,80Hz), δ = 2,02 ppm (dquint, 2H, CH₂CH₂CH₂OP, J=6,33 ; 1,02Hz), δ = 2,72 ppm (s, 3H, CH₃NCH₂CH₂CH₂N₃), δ = 2,75ppm (s, 3H, CH₃NCH₂CH₂CH OP), δ = 3,24 ppm (t, 4H, NCH₂CH₂, J = 6,86Hz), δ = 3,35 ppm (t, 2H, NCH₂CH₂CH₂N₃, J = 6,75Hz), δ = 4,35 ppm (quad, 2H, NCH₂CH₂CH₂OP, J = 6,68Hz), δ = 7,16-7,37 ppm (m, 10H, Aromatiques). RMN ¹³C (CDCl₃, 25°C, 75 MHz) : δ =26,95 ppm (s, NCH₂CH₂CH₂N₃), δ = 28,32 ppm (d, NCH₂CH₂CH₂OP, J =7 ,50Hz), δ = 37,98 ppm (N3CH₂CH₂CH₂NCH₃), δ = 38,15 ppm (POCH₂CH₂CH₂NCH₃), δ = 49,41 ppm (NCH₂CH₂CH₂N₃), δ = 51,57 ppm (NCH₂CH₂CH₂OP), δ = 67,54 ppm (d, NCH₂CH₂CH₂OP, J = 6,75 Hz), δ = 120,20 ppm (d, POC(CH)₂(CH)₂C, J = 4,50 Hz), δ = 125,49 ppm (POC(CH)₂(CH)₂C), δ = 129,93 ppm (POC(CH)₂(CH)₂C), δ = 150,71 ppm (d, POC(CH)₂(CH)₂C, J = 7,50Hz).

RMN ³¹P {1H} (CDCl₃, 25°C, 120 MHz) : δ = -11,91 ppm (CH₂OP(OPh)₂).

HRMS (ESI⁺) : [M+H]⁺ m/z = 462,2013 (théorique); 462,2017 (mesurée); -0,8 ppm.

[M+Na]⁺ m/z = 484,1833 (théorique); 484,1833 (mesurée); 0 ppm.

IR (Golden Gate) : v (cm-1) = 2963, 2851, 2094, 1590, 1488, 1286, 1188, 939.

### Exemple 2'b

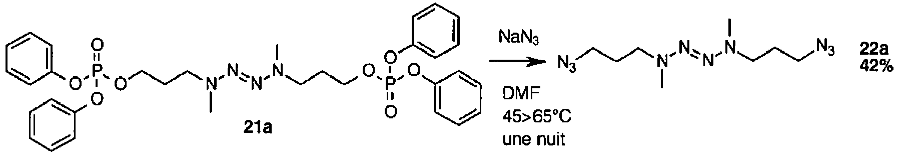

### Synthèse du (E)-1,4-bis(3-azidopropyl)-1,4-diméthyltétrazène (22a)

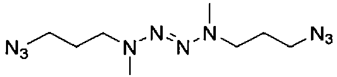

### « Méthode 1 » : Azidation du tétrazène 21a

Le tétrazène **21a** (169 mg, 0,83 mmol) a été introduit dans un ballon monocol de 25 mL et dilué dans 3 mL de DMF. L'azoture de sodium (82,16 mg, 1,26 mmol) a ensuite été ajouté par portions et le milieu réactionnel a été chauffé à 45°C durant une nuit. Le lendemain matin la température a été réhaussée à 65°C et la réaction s'est poursuivie durant 6 h. La réaction a été stoppée en ajoutant 25 mL d'une solution saturée de NaCl. La phase aqueuse a ensuite été extraite avec de l'éther diéthylique (3*25mL). Les phases ont été séparées et la phase organique a été séchée sur MgSO₄ puis le solvant a été évaporé sous pression réduite. Une purification sur colonne de silice avec un éluant CH₂Cl₂ a permis d'obtenir le produit **22a** sous forme d'un liquide incolore (27 mg, rendement 42%).

Le tétrazène di-azidé obtenu correspond à celui préparé dans l'exemple ci-après. Voir, dans ledit exemple ci-après ses caractéristiques.

### Exemple 2'c

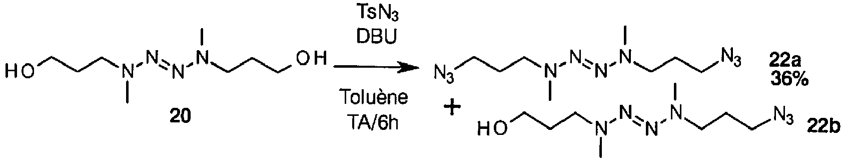

### Synthèse du (E)-1,4-bis(3-azidopropyl)-1,4-diméthyltétrazène (22a) et du (E)-1-(3-hydroxypropyl))-4-(3-azidopropyl)-1,4-diméthyltétrazène (22b)

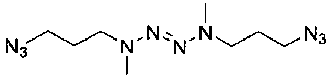

### « Méthode 2 » : Azidation du tétrazène 20

Le tétrazène **20** (358,8 mg, 1,76 mmol) a été introduit dans un ballon monocol de 25 mL et dilué dans 2 mL de toluène. Le TsN₃ (azoture de tosyle) (732,5 mg, 3,71 mmol) a été ajouté puis le DBU (1,8-DiazaBicyclo[5.4.0]Undéc-7-ène) (560 mL, 3,74 mmol) au goutte à goutte et la réaction s'est poursuivi durant 6 h à température ambiante. Le solvant a été ensuite évaporé sous pression réduite et le résidu a été dilué dans 5 mL de dichlorométhane. Une purification sur colonne de silice avec un éluant CH₂Cl₂/MeOH (Gradient 0-10% sur 20 minutes) a permis d'isoler les produits **22a et 22b** : le produit **22a** sous la forme d'un liquide incolore (161 mg, rendement 36 %), le produit **22b** sous la forme d'un liquide jaune (110 mg, 27%) (avec un temps de rétention plus élevé que 22a).

### Caractérisation 22a (exemples 2'b et 2'c)

RMN ¹H (CDCl₃, 25°C, 300 MHz) : δ = 1,92 ppm (quint, 4H, CH₂C***H**₂*CH₂N₃, J = 6,80 Hz), δ = 2,80 ppm (s, 6H, NC***H**₃*), δ = 3,28 ppm (t, 4H, NC***H**₂*CH₂CH₂N₃, J = 6,84 Hz), δ = 3,39 ppm (t, 4H, NCH₂CH₂C***H₂***N₃, J = 6,77Hz).

RMN ¹³C (CDCl₃, 25°C, 300 MHz) : δ = 26,93 ppm (NCH₂***C***H₂CH₂N₃), δ = 52,54 ppm (N***C***H₂CH₂CH₂N₃), δ = 49,41 ppm (NCH₂CH₂***C***H₂N₃), δ = 38,02 ppm (N***C***H₃).

HRMS (ESI⁺) : [M+H]⁺ m/z = 255,1781 (théorique); 255,1789 (mesurée); 2,8 ppm.

[M+Na]⁺ m/z = 277,1602 (théorique); 277,1608 (mesurée); 3,2 ppm.

IR (Golden Gate, rel. int.) : v (cm⁻¹) = 2962, 2853, 2089, 1462, 1253, 1007.

DSC (25°C à 350°C ; 2°C/min) : T_{d} (pic onset) = 119°C (-942J/g).

UV : λₘₐₓ = 280 nm

### Caractérisation 22b (exemple 2'c)

RMN ¹H (CDCl₃, 25°C, 300 MHz) : δ = 1,86 ppm (quint, 2H, CH₂CH₂CH₂N₃, J = 6,19 Hz), δ = 1,89 ppm (quint, 2H, CH₂CH₂CH₂OH, J = 6,83 Hz), δ = 2,79 ppm (s, 6H, NCH₃), δ = 3,27 ppm (t, 2H, NCH₂CH₂CH₂N₃, J = 6,90 Hz), δ = 3,32 ppm (t, 2H, NCH₂CH₂CH₂OH, J = 6,60 Hz), δ = 3,36 ppm (t, 2H, NCH₂CH₂CH₂N₃, J = 6,75 Hz), δ = 3,72 ppm (t, 2H, NCH₂CH₂CH₂OH, J = 5,91 Hz).

RMN ¹³C (CDCl₃, 25°C, 75 MHz) : δ = 26,94 ppm (NCH₂CH₂CH₂N₃), δ = 30,05 ppm (NCH₂CH₂CH₂OH), δ = 37,97-38,32 ppm (N*C*H₃), δ = 49,39 ppm (NCH₂CH₂CH₂N₃), δ = 52,55 ppm (NCH₂CH₂CH₂N₃), δ = 52,90 ppm (NCH₂CH₂CH₂OH), δ = 61,57 ppm (NCH₂CH₂CH₂OH).

HRMS (ESI⁺) : [M+H]⁺ m/z = 230,1724 (théorique); 230,1715 (mesurée); 3,9ppm.

[M+Na]⁺ m/z = 252,1543 (théorique); 252,1539 (mesurée); 1,5 ppm.

### Exemple 3'

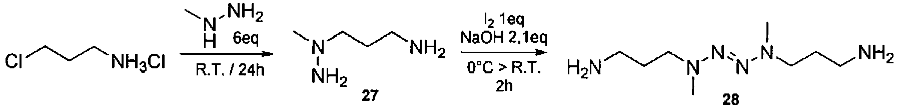

### Synthèse de la 1-(3-aminopropyl)-1-méthylhydrazine (27)

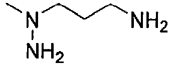

Dans un ballon monocol de 25 mL ont été introduits 5 mL de monométhylhydrazine (94,96 mmol) puis une atmosphère inerte d'argon a été instaurée dans le milieu. Le ballon a été plongé dans un bain d'eau à température ambiante afin de drainer une exothermicité trop importante au cours de la réaction. Le chlorhydrate de 3-chloropropylamine (2,00 g, 15,38 mmol) a alors été ajouté par portions et la réaction s'est poursuivi 24 h à température ambiante. Le milieu a ensuite été basifié à l'aide d'une solution aqueuse saturée d'hydroxyde de sodium (25 mL) puis les espèces volatiles ont été évacuées sous pression réduite. L'hydrazine a été séparée de la phase aqueuse par démixtion et les sels ont été précipités dans l'acétonitrile (50 mL). Une filtration suivie d'une évaporation a permis d'obtenir le produit **27** sous la forme d'un liquide orangé (1056,6 mg ; 67%).

RMN ¹H (CDCl₃, 25°C, 300 MHz) : δ = 1,65 ppm (quint, 2H, NCH₂C***H**₂*CH₂NH₂, J = 6,95Hz), δ = 2,44 ppm (s, 3H, NC***H₃***), δ = 2,47 ppm (t, 2H, NCH₂CH₂C***H₂***NH₂, J=7,02Hz), δ = 2,75 ppm (t, 2H, NC***H₂***CH₂CH₂NH₂, J = 6,86Hz).

RMN ¹³C (CDCl₃, 25°C, 75 MHz) : δ =31,46 ppm (NCH₂***C***H₂CH₂NH₂), δ = 40,52 ppm (NCH₂CH₂***C***H₂NH₂), δ = 49,99 ppm (N***C***H₃), δ = 61,28 ppm (N***C***H₂CH₂CH₂NH₂).

On rappelle à toutes fins utiles que ce produit est commercialement disponible.

### Synthèse du (E)-1,4-bis(3-aminopropyl)-1,4-diméthyltétrazène (28)

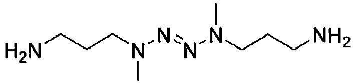

Dans un ballon monocol de 100 mL l'hydrazine **28** (1056,6 mg, 10,24 mmol) a été introduite et diluée dans 20 mL d'éthanol anhydre. Le milieu a été refroidi à 0°C au moyen d'un bain de glace puis l'hydroxyde de sodium (861,5 mg, 21,51 mmol) a été ajouté. Le diiode (2,60 g, 10,24 mmol) a alors été additionné par portion puis le milieu a été retiré du bain de glace et la réaction s'est poursuivie durant 2 h à température ambiante. Une solution aqueuse saturée de thiosulfate de sodium (5 mL) a ensuite été ajoutée afin de neutraliser le diiode restant puis le solvant a été évaporé sous pression réduite. Les sels ont été précipités dans l'acétonitrile (25 mL), filtrés et le solvant a été évaporé sous pression réduite. Une purification sur colonne de silice (DCM/MeOH/Et₃N 90/9/1 v/v) a permis d'obtenir le produit **28.**

### Exemple 4'

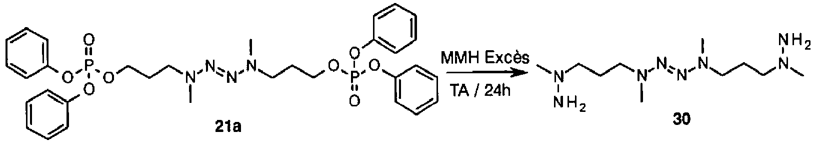

### Synthèse du (E)-1,4-bis(3-(1-méthylhydrazinyl)propyl)-1,4-diméthyltétrazène (30)

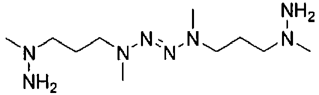

Dans un ballon monocol de 50 mL le tétrazène **21a** (200 mg, 0,30 mmol) a été introduit puis le milieu a été placé sous atmosphère inerte d'argon. La monométhylhydrazine (3 mL, 57,0 mmol) a ensuite été ajoutée et la réaction s'est poursuivie 24 h à température ambiante. La MMH a été évaporée sous pression réduite puis le résidu a été dissout dans 10 mL d'une solution saturée de NaHCO₃. La phase aqueuse a alors été extraite en utilisant du dichlorométhane (4*25 mL) et les phases organiques ont regroupées, séchées sur sulfate de magnésium et évaporées sous pression réduite. Le produit **30** a été isolé en quantités suffisantes pour effectuer des analyses RMN et HRMS.

RMN ¹H (CDCl₃, 25°C, 300MHz) : δ = 1,83 ppm (quint, 4H, NCH₂C***H₂***CH₂NMeNH₂, J = 7,25Hz), δ = 2,47 ppm (s, 6H, NNH₂C***H₃***), δ = 2,49 ppm (t, 4H, NCH₂CH₂C***H₂***NMeNH₂, J = 7,05Hz), δ = 2,76 ppm (s, 6H, NC***H₃***), δ = 3,24 ppm (t, 4H, NC***H₂***CH₂CH₂NMeNH₂, J = 7,23Hz).

HRMS (ESI⁺) : [M+H]⁺ m/z = 261,2510 (théorique); 261,2508 (mesurée) ; 0,5 ppm.

[M+Na]⁺ m/z = 283,2329 (théorique); 283,2339 (mesurée); -3,4 ppm.

### Exemple 5'

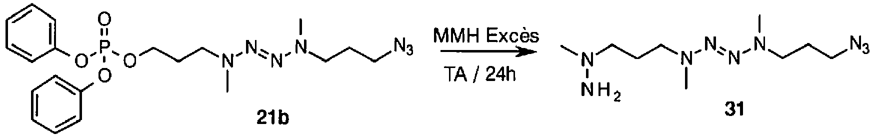

### Synthèse du (E)-1-(3-azidopropyl)-4-(3-(1-méthylhydrazinyl)propyl)-1,4-diméthyltétrazène (31)

Du tétrazène 21b (703,5 mg, 1,52 mmol) a été introduit dans un ballon monocol de 50 mL. L'intérieur dudit ballon a alors été mis sous atmosphère inerte d'argon. La monométhylhydrazine (3 mL, 57,0 mmol) a ensuite été ajoutée et la réaction poursuivie 24 h à température ambiante. A l'issue de ces 24 h, 10 mL d'une solution aqueuse saturée de NaOH ont été ajoutés pour basifier le milieu réactionnel et les composés volatils ont été évaporés sous pression réduite. La phase aqueuse a alors été extraite en utilisant du dichlorométhane (4*25 mL) et les phases organiques regroupées ont été séchées sur sulfate de magnésium et évaporées sous pression réduite jusqu'à l'obtention d'un liquide incolore (298,8 mg, 76%).

¹H NMR (CDCl₃, 25°C, 300 MHz): δ (ppm) = 1,83 (quint., 2H, NCH₂CH₂CH₂NMeNH₂, J = 7,22 Hz), 1,90 (quint., 2H, NCH₂CH₂CH₂N₃, J = 6,83 Hz), 2,47 (s, 3H, NMeNH₂), 2,48 (t, 2H, NCH₂CH₂CH₂NMeNH₂, J = 7,25 Hz), 2,76 (s, 3H, MeNN=NNMe), 2,77 (s, 3H, MeNN=NNMe), 3,23 (t, 2H, NCH₂CH₂CH₂NMeNH₂, J = 6,78 Hz), 3,26 (t, 2H, NCH₂CH₂CH₂N₃, J = 6,75 Hz), 3,36 (t, 2H, NCH₂CH₂CH₂N₃, J = 6,80 Hz).

### Tétrazènes β,γ-fonctionnalisés de formule (le)

### Exemple 1"

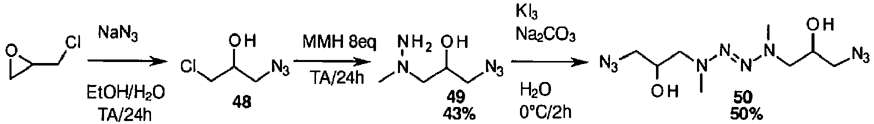

### Synthèse du 1-azido-3-chloropropan-2-ol (48) (d'après Gharakanian et al., Biomacromolecules, 2015, 16 (6), 1802-1806)

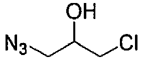

L'azoture de sodium (1,97 g, 30,25 mmol) a été introduit dans un ballon de 25 mL et dilué dans un mélange éthanol/eau (25/75, v/v). Le milieu a ensuite été acidifié en utilisant de l'acide acétique (1,72 mL, 30,0 mmol). L'épichlorohydrine (ECH) (1,96 mL, 25 mmol) a alors été additionnée sur 15 minutes au moyen d'un pousse seringue et la réaction s'est poursuivie à température ambiante durant 24 h. Le milieu réactionnel a été traité avec 20 mL d'une solution saturée de chlorure de sodium puis la phase aqueuse a été extraite avec de l'acétate d'éthyle (3*25 mL). Les phases organiques ont été regroupées, séchées sur sulfate de magnésium et le solvant a été évacué sous pression réduite.

RMN ¹H (CDCl₃, 25°C, 300 MHz) : δ = 3,47 ppm (d, 2H, ClCH₂CHOHC***H₂***N₃, J = 5,34Hz), δ = 3,61 ppm (dd, 2H, ClC***H₂***CHOHCH₂N₃, J₁ = 2,37Hz, J₂ = 5,01 Hz), δ = 4,13 ppm (quint, 1H, ClCH₂C***H***OHCH₂N₃, J = 5,39Hz).

### Synthèse du 1-azido-3-(1-méthylhydrazinyl)propan-2-ol (49)

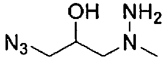

La monométhylhydrazine (880 L, 16,7 mmol) a été introduite dans un ballon monocol de 10 mL et une atmosphère inerte γ a été établie. L'azoture **(48)** a ensuite été ajouté au goutte à goutte et la réaction s'est poursuivie à température ambiante durant 24 h. Le milieu a été traité avec quelques gouttes d'une solution aqueuse saturée de NaOH puis dilué avec 5 mL d'acétonitrile. Une filtration des sels suivie d'une évaporation du solvant et de la monométhylhydrazine sous pression réduite a permis d'obtenir le produit sous la forme d'un liquide visqueux jaune pâle (260,2 mg, rendement 43%).

RMN ¹H (CDCl₃, 25°C, 300 MHz) : δ = 2,54 ppm (s, 3H, NC***H₃***), δ = 2,46-2,63 ppm (m, 2H, NC***H*₂**CHOHCH₂N₃), δ = 4,01 ppm (m, 1H, ClCH₂C***H***OHCH₂N₃), δ = 3.33 ppm (t, 2H, NCH₂CHOHC***H₂***N₃, J = 5,73Hz).

### Synthèse du (E)-1,4-bis(3-azido-2-hydroxypropyl)-1,4-diméthyltétrazène (50)

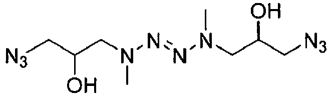

L'hydrazine **49** (260,2 mg, 1,79 mmol) a été introduite dans un ballon monocol de 50 mL et diluée dans 5,5 mL d'eau distillée (C=0,25 M). Le carbonate de sodium (210 mg, 1,98 mmol) a ensuite été ajouté et le milieu a été refroidi à 0°C en utilisant un bain de glace. Une solution aqueuse de KI₃ d'une concentration de 1,33M (1,48 mL, 1,97 mmol) a alors été additionnée au goutte à goutte. La température du milieu a été maintenue en dessous de 5°C durant cet ajout. Le bain de glace a ensuite été retiré et la réaction s'est poursuivie à température ambiante pendant 2h. Le milieu réactionnel a été extrait avec du dichlorométhane (3*25 mL) puis les phases organiques ont été regroupées, séchées sur sulfate de magnésium et le solvant a été évaporé sous pression réduite. Une purification sur colonne de silice (CH₂Cl₂/MeOH 1% v/v) a permis d'obtenir le produit pur sous la forme d'un liquide incolore (127,4 mg, rendement 50%).

RMN ¹H (CDCl₃, 25°C, 300 MHz) : δ = 4,11 ppm (quint, 2H, CH₂C***H**O*HCH₂N₃, J = 5,48Hz), δ = 2,87 ppm (s, 6H, NC***H₃***), δ = 3,20-3,47 ppm (m, 8H, NC***H₂***CHOHC***H₂***N₃).

RMN ¹³C (CDCl₃, 25°C, 300 MHz) : δ = 69,36 ppm (NCH₂***C***HOHCH₂N₃), δ = 58,83 ppm (N***C***H₂CHOHCH₂N₃), δ = 54,46 ppm (NCH₂CHOH***C***H₂N₃), δ = 39,57 ppm (N***C***H₃).

HRMS (ESI⁺) : [M+Na]⁺ m/z = 309,1506 (théorique); 309,1502 (mesurée); 1,4 ppm.

[2M+Na]⁺ m/z = 595,3121 (théorique); 595,3111 (mesurée); 1,6 ppm.

### IR (Golden Gate, rel. int.) : v (cm⁻¹) = 3394, 2923, 2093, 1465, 1438, 1265, 1008.

DSC (25°C à 350°C ; 2°C/min) : T_{d} (pic onset) = 116°C (-1156J/g).

UV/Vis : λₘₐₓ = 281 nm

## Revendications

1. 1,4-Diméthyltétrazène choisi parmi :
- les 1,4-diméthyltétrazènes β-fonctionnalisés de formule (Ia) :
**R1-CH₂-N(CH₃)-N=N-N(CH₃)-CH₂-R2** **(Ia)**
dans laquelle
R1 = R2 = **-CH₂Cl, -CH₂N₃, -CH₂NH₂, -CH₂N(CH₃)(NH₂), -CH=CH₂,** ou **-CH₂NCO** ; ou
R1 et R2, identiques, renfermant un groupe éther, sont choisis parmi les couples ci-après:
R1 = R2 = **-CH₂O-CH₂-CH=CH₂,**
R1 = R2 = **-CH₂O-CH₂-C≡CH,** et
R1 = R2 = **-CH₂O-CH₂-C≡N** ;
- les 1,4-diméthyltétrazènes γ-fonctionnalisés de formule (Ib) :
**R3-CH₂-CH₂-N(CH₃)-N=N-N(CH₃)-CH₂-CH₂-R4** **(Ib)**
dans laquelle
R3 = R4 = **-CH₂OH, -CH₂N₃, -CH₂NH₂, -CH₂N(CH₃)(NH₂), -CH=CH₂, -CH₂NCO, -CH₂CN ou -CH₂ONO₂,** ou
R3 = **-CH₂OH** et R4 = **-CH₂N₃,** ou
R3 = **-CH₂CN** et R4 = **-CH₂N₃,** ou
R3 = **-CH₂N₃** et R4 = **-CH₂N(CH₃)(NH₂),** ou
R3 et R4, identiques ou différents, l'un au moins d'entre eux renfermant un groupe éther, sont choisis parmi les couples ci-après:
R3 = R4 = **-CH₂O-CH₂-CH=CH₂**
R3 = R4 = **-CH₂O-CH₂-C≡CH**
R3 = R4 = **-CH₂O-CH₂-C≡N**
R3 = **-CH₂O-CH₂-CH=CH₂** et R4 = **-CH₂N₃**
R3 = **-CH₂O-CH₂-C≡CH** et R4 = **-CH₂N₃,** et
R3 = **-CH₂O-CH₂-C≡N** et R4 = **-CH₂N₃** ;
et
- les 1,4-diméthyltétrazènes β, γ-fonctionnalisés de formule (Ic) :
**R5-CHOH-CH₂-N(CH₃)-N=N-N(CH₃)-CH₂-CHOH-R5** **(Ic)**
dans laquelle
R5 = **-CH₂N₃** ou **-CH₂CN.**

2. Procédé pour la préparation des 1,4-diméthyltétrazènes selon la revendication 1, qui comprend :
- la mise à disposition d'une hydrazine précurseur, puis,
- par oxydation, la dimérisation de celle-ci.

3. Procédé selon la revendication 2, dans lequel l'oxydation de l'hydrazine précurseur est mise en oeuvre a) avec le diiode en solution dans un solvant organique, notamment choisi parmi l'éthanol et l'éther diéthylique et consistant avantageusement en l'éthanol, ou b) avec KI₃ en solution aqueuse, ou c) avec la monochloramine.

4. Procédé selon la revendication 2 ou 3, qui comprend, pour la préparation des 1,4-diméthyltétrazènes β-fonctionnalisés de formule **(Ia)** :
- la mise à disposition d'une hydrazine précurseur choisie parmi la 1-(2-chloroéthyl)-1-méthylhydrazine, sous forme libre et/ou chlorhydratée, la 1-(2-azidoéthyl)-1-méthylhydrazine, la 1-(2-aminoéthyl)-1-méthylhydrazine, et la 1-allyl-1-méthylhydrazine, puis, par oxydation, l'obtention du dimère correspondant, tétrazène de formule **(Ia)** dans laquelle, respectivement, R1 = R2 = **-CH₂Cl, -CH₂N₃, -CH₂NH₂** ou **-CH=CH₂;** ou
- l'obtention du *(E)*-1,4-bis(2-hydroxyéthyl)-1,4-diméthyltétrazène et l'alkylation de ses fonctions hydroxyle pour l'obtention des diéthers symétriques ; et
- possiblement, la conversion, par substitution nucléophile avec de la monométhylhydrazine, du tétrazène de formule **(Ia)** dans laquelle R1 = R2 = **-CH₂Cl** en le tétrazène de formule **(Ia)** dans laquelle R1 = R2 = **-CH₂N(CH₃)(NH₂)** ou la conversion du tétrazène de formule **(Ia)** dans laquelle R1 = R2 = **-CH₂NH₂,** avantageusement par réaction de ses fonctions amine avec du 1,1'-carbonyldiimidazole, en le tétrazène de formule **(Ia)** dans laquelle R1 = R2 = **-CH₂NCO.**

5. Procédé selon la revendication 4, dans lequel la 1-(2-azidoéthyl)-1-méthylhydrazine est obtenue par azidation en milieu aqueux de la 1-(2-chloroéthyl)-1-méthylhydrazine, sous forme libre.

6. Procédé selon la revendication 4 ou 5, dans lequel la 1-(2-chloroéthyl)-1-méthylhydrazine et la 1-(2-azidoéthyl)-1-méthylhydrazine sont oxydées avec la monochloramine, et dans lequel la 1-allyl-1-méthylhydrazine est oxydée avec le diiode en solution dans l'éther diéthylique.

7. Procédé selon la revendication 2 ou 3, qui comprend, pour la préparation des 1,4-diméthyltétrazènes γ-fonctionnalisés de formule **(Ib)** :
- la mise à disposition d'une hydrazine précurseur choisie parmi la 1-(3-hydroxypropyl)-1-méthylhydrazine, la 1-(3-aminopropyl)-1-méthylhydrazine et la 1-but-1-ènyl-1-méthylhydrazine, puis, par oxydation, l'obtention du dimère correspondant, tétrazène de formule **(Ib)** dans laquelle, respectivement, R3 = R4 = **-CH₂OH, -CH₂NH₂** ou **-CH=CH₂ ;**
- possiblement, la conversion du tétrazène de formule **(Ib)** dans laquelle R3 = R4 = **-CH₂NH₂,** avantageusement par réaction de ses fonctions amine avec du 1,1'-carbonyl-dimidazole, en le tétrazène de formule **(Ib)** dans laquelle R3 = R4 = **-CH₂NCO** ;
- possiblement, l'utilisation du tétrazène de formule **(Ib)** dans laquelle R3 = R4 = **-CH₂OH** :
+ pour obtenir, par alkylation de ses deux fonctions hydroxyle, un de ses di-éthers symétriques de formule **(Ib),** ou
+ pour obtenir, par nitration, avec avantageusement le tétrafluoroborate de nitronium, le tétrazène de formule **(Ib)** dans laquelle R3 = R4 = **-CH₂ONO₂,** ou
+ pour obtenir, par azidation, avantageusement en utilisant l'azoture de tosyle en présence d'une base faible telle le 1,8-diazabicyclo[5.4.0]undéc-7-ène, le tétrazène de formule **(Ib)** dans laquelle R3 = R4 = **-CH₂N₃** et le tétrazène de formule **(Ib)** dans laquelle R3 **=-CH₂OH** et R4 = **-CH₂N₃,** la fonction hydroxyle de ce dernier pouvant être alkylée pour l'obtention des monoéthers de formule **(Ib)** ; ou
+ pour obtenir, par action de l'azoture de diphénylphosphoryle en présence d'une base faible telle le 1,8-diazabicyclo[5.4.0]undéc-7-ène,
le *(E)*-1,4-bis(3-(diphénylphosphoryl)propyl)-1,4-diméthyltétrazène, intermédiaire convenant à l'obtention, selon une autre voie d'azidation, dudit tétrazène de formule **(Ib)** dans laquelle R3 = R4 = **-CH₂N₃,** ainsi qu'à l'obtention, par cyanation, du tétrazène de formule **(Ib)** dans laquelle R3 = R4 = **-CH₂CN** et à l'obtention, par substitution nucléophile avec de la monométhylhydrazine, du tétrazène de formule **(Ib)** dans laquelle R3 = R4 = **-CH₂N(CH₃)(NH₂),** ou
le *(E)*-1-(3-(diphénylphosphoryl)-4-(3-azidopropyl)-1,4-diméthyltétrazène, intermédiaire convenant à l'obtention, par cyanation, du tétrazène de formule **(Ib)** dans laquelle R3 = **-CH₂N₃** et R4 = **-CH₂CN** et à l'obtention, par substitution nucléophile avec de la monométhylhydrazine, du tétrazène de formule **(Ib)** dans laquelle R3 = **-CH₂N₃** et R4 = **-CH₂N(CH₃)(NH₂).**

8. Procédé selon la revendication 7, dans lequel la 1-(3-hydroxypropyl)-1-méthylhydrazine est obtenue par condensation d'un excès de monométhylhydrazine (MMH) sur du 3-chloropropan-1-ol.

9. Procédé selon la revendication 7 ou 8, dans lequel la 1-(3-hydroxypropyl)-1-méthylhydrazine est oxydée avec KI₃ en solution aqueuse ou avec le diiode en solution dans l'éthanol, avantageusement avec le diiode en solution dans l'éthanol, et dans lequel la 1-but-1-ènyl-1-méthylhydrazine est oxydée avec le diiode en solution dans l'éther diéthylique.

10. Procédé selon la revendication 2 ou 3, qui comprend, pour la préparation des 1,4-diméthyltétrazènes β,γ-fonctionnalisés de formule **(Ic)** :
- la mise à disposition du 1-azido-3-(1-méthylhydrazinyl)propan-2-ol ou du 1-cyano-3-(1-méthylhydrazinyl)propan-2-ol, puis
- par oxydation, la dimérisation, qui donne le tétrazène attendu, respectivement diazido ou dicyané.

11. Procédé selon la revendication 10, dans lequel ladite mise à disposition du 1-azido-3-(1-méthylhydrazinyl)propan-2-ol ou du 1-cyano-3-(1-méthylhydrazinyl)propan-2-ol comprend :
- l'ouverture de l'épichlorhydrine par, respectivement, le nitrure de sodium ou le cyanure de potassium, et
- la condensation du produit résultant avec la monométhylhydrazine (MMH), pour, respectivement, l'obtention dudit 1-azido-3-(1-méthylhydrazinyl)propan-2-ol ou dudit 1-cyano-3-(1-méthylhydrazinyl)propan-2-ol.

12. Procédé selon la revendication 10 ou 11, dans lequel le 1-azido-3-(1-méthylhydrazinyl)propan-2-ol ou le 1-cyano-3-(1-méthylhydrazinyl)propan-2-ol sont oxydés avec KI₃ en solution aqueuse.

13. Procédé pour la préparation du *(E)*-1,4-bis(2-hydroxyéthyl)-1,4-diméthyltétrazène, qui comprend :
- la condensation d'un excès de monométhylhydrazine sur du 2-chloroéthanol pour obtenir la 1-(2-hydroxyéthyl)-1-méthylhydrazine ; puis
- la dimérisation de l'hydrazine ainsi obtenue par oxydation.

14. Procédé selon la revendication 13, dans lequel l'oxydation de ladite 1-(2-hydroxyéthyl)-1-méthylhydrazine est mise en oeuvre a) avec le diiode en solution dans un solvant organique, notamment choisi parmi l'éthanol et l'éther diéthylique et consistant avantageusement en l'éthanol, ou b) avec KI₃ en solution aqueuse, ou c) avec la monochloramine ; avantageusement l'oxydation est mise en oeuvre avec le diiode en solution dans l'éthanol.

15. Intermédiaire, utile à la préparation d'au moins l'un des tétrazènes selon la revendication 1, ledit intermédiaire étant choisi parmi :
- la 1-(2-azidoéthyl)-1-méthylhydrazine ;
- le *(E)*-1,4-bis(3-(diphénylphosphoryl)propyl)-1,4-diméthyltétrazène ;
- le *(E)*-1-(3-(diphénylphosphoryl)-4-(3-azidopropyl)-1,4-diméthyltétrazène ;
- le 1-azido-3-(1-méthylhydrazinyl)propan-2-ol ; et
- le 1-cyano-3-(1-méthylhydrazinyl)propan-2-ol.

## Patentansprüche

1. 1,4-Dimethyltetrazen, ausgewählt aus:
- β-funktionalisierte 1,4-Dimethyltetrazene der Formel (Ia):
R1-CH₂-N(CH₃)-N=N-N(CH₃)-CH₂-R2 (Ia)
in der
R1 = R2 = **-CH₂Cl, -CH₂N₃, -CH₂NH₂, -CH₂N(CH₃)(NH₂), -CH=CH₂,** oder **-CH₂NCO;** oder
R1 und R2, die identisch sind und eine Ethergruppe enthalten, aus den folgenden Paaren ausgewählt sind:
R1 = R2 = -CH₂O-CH₂-CH=CH₂,
R1 = R2 = -CH₂O-CH₂-C≡CH, und
R1 = R2 = -CH₂O-CH₂-C≡N;
- γ-funktionalisierte 1,4-Dimethyltetrazene der Formel (Ib):
R3-CH₂-CH₂-N(CH₃)-N=N-N(CH₃)-CH₂-CH₂-R4 (Ib)
in der
R3 = R4 = -CH₂OH, -CH₂N₃, -CH₂NH₂, -CH₂N(CH₃)(NH₂), -CH=CH₂, -CH₂NCO, -CH₂CN oder -CH₂ONO₂, oder
R3 = -CH₂OH und R4 = -CH₂N₃, oder
R3 = -CH₂CN und R4 = -CH₂N₃, oder
R3 = -CH₂N₃ und R4 = -CH₂N(CH₃)(NH₂), oder
R3 und R4, die gleich oder verschieden sind, wobei mindestens einer von ihnen eine Ethergruppe enthält, aus den folgenden Paaren ausgewählt sind:
R3 = R4 = -CH₂O-CH₂-CH=CH₂
R3 = R4 = -CH₂O-CH₂-C≡CH
R3 = R4 = -CH₂O-CH₂-C≡N
R3 = -CH₂O-CH₂-CH=CH₂ und R4 = -CH₂N₃
R3 = -CH₂O-CH₂-C≡CH und R4 = -CH₂N₃, und
R3 = -CH₂O-CH₂-C≡N und R4 = -CH₂N₃;
und
- β,γ-funktionalisierte 1,4-Dimethyltetrazene der Formel (Ic):
R5-CHOH-CH₂-N(CH₃)-N=N-N(CH₃)-CH₂-CHOH-R5 (Ic)
in der
R5 = -CH₂N₃ oder -CH₂CN.

2. Verfahren zur Herstellung von 1,4-Dimethyltetrazene nach Anspruch 1, das umfasst:
- Bereitstellen eines Hydrazinvorläufers und dann,
- durch Oxidation die Dimerisierung desselben.

3. Verfahren nach Anspruch 2, bei dem die Oxidation des Hydrazinvorläufers durchgeführt wird a) mit Diod in Lösung in einem organischen Lösungsmittel, das insbesondere aus Ethanol und Diethylether ausgewählt wird und vorteilhafterweise aus Ethanol besteht, oder b) mit KI₃ in wässriger Lösung, oder c) mit Monochloramin.

4. Verfahren nach Anspruch 2 oder 3, das zur Herstellung der β-funktionalisierten 1,4-Dimethyltetrazene der Formel (Ia) Folgendes umfasst:
- Bereitstellung eines Hydrazinvorläufers, ausgewählt aus 1-(2-Chlorethyl)-1-methylhydrazin, in freier und/oder hydrochlorierter Form, 1-(2-Azidoethyl)-1-methylhydrazin, 1-(2-Aminoethyl)-1-methylhydrazin, und 1-Allyl-1-methylhydrazin, und dann durch Oxidation die Gewinnung des entsprechenden Dimer, das ein Tetrazen der Formel (Ia) ist, worin jeweils R1 = R2 = -CH₂Cl, -CH₂N₃, -CH₂NH₂ oder -CH=CH₂; oder
- die Gewinnung von (E)-1,4-Bis(2-hydroxyethyl)-1,4-dimethyltetrazol und die Alkylierung seiner Hydroxylfunktionen zur Gewinnung der symmetrischen Diether; und
- möglicherweise Umwandlung, durch nukleophile Substitution mit Monomethylhydrazin, des Tetrazens der Formel (Ia) worin R1 = R2 = -CH₂Cl in das Tetrazen der Formel (Ia) worin R1 = R2 = -CH₂N(CH₃)(NH₂) oder die Umwandlung des Tetrazens der Formel (Ia) worin R1 = R2 = -CH₂NH₂, vorteilhafterweise durch Umsetzung seiner Aminfunktionen mit 1,1'-Carbonyldiimidazol, in das Tetrazen der Formel (Ia) worin R1 = R2 = -CH₂NCO.

5. Verfahren nach Anspruch 4, bei dem 1-(2-Azidoethyl)-1-methylhydrazin durch Azidierung von 1-(2-Chlorethyl)-1-methylhydrazin in freier Form in wässrigem Medium erhalten wird.

6. Verfahren nach Anspruch 4 oder 5, bei dem 1-(2-Chlorethyl)-1-methylhydrazin und 1-(2-Azidoethyl)-1-methylhydrazin mit Monochloramin oxidiert werden und bei dem 1-Allyl-1-methylhydrazin mit Diod in Diethyletherlösung oxidiert wird.

7. Verfahren nach Anspruch 2 oder 3, das zur Herstellung der γ-funktionalisierten 1,4-Dimethyltetrazene der Formel (Ib) Folgendes umfasst:
- Bereitstellung eines Hydrazinvorläufers, ausgewählt aus 1-(3-Hydroxypropyl)-1-methylhydrazin, 1-(3-Aminopropyl)-1-methylhydrazin und 1-But-1-enyl-1-methylhydrazin, und dann durch Oxidation die Gewinnung des entsprechenden Dimers, das ein Tetrazen der Formel (Ib) ist worin jeweils R3 = R4 = -CH₂OH, -CH₂NH₂ oder -CH=CH₂;
- möglicherweise Umwandlung des Tetrazens der Formel (Ib), worin R3 = R4 = -CH₂NH₂, vorteilhafterweise durch Umsetzung seiner Aminfunktionen mit 1,1'-Carbonyl-Dimidazol, in das Tetrazen der Formel (Ib), worin R3 = R4 = -CH₂NCO;
- möglicherweise die Verwendung des Tetrazens der Formel (Ib), in der R3 = R4 = -CH₂OH:
+ um durch Alkylierung seiner beiden Hydroxylfunktionen einen seiner symmetrischen DiEther der Formel (Ib) zu erhalten, oder
+ um durch Nitrierung, vorteilhafterweise mit Nitroniumtetrafluoroborat, das Tetrazen der Formel (Ib) zu erhalten, in der R3 = R4 = -CH₂ONO₂, oder
+ um durch Azidierung, vorteilhafterweise unter Verwendung von Tosylazid in Gegenwart einer schwachen Base wie 1,8-Diazabicyclo[5.4.0]undec-7-en, das Tetrazen der Formel (Ib) zu erhalten, in der R3 = R4 = -CH₂N₃ und das Tetrazen der Formel (Ib), in der R3 = -CH₂OH und R4 = -CH₂N₃, wobei die Hydroxylfunktion des letzteren alkyliert werden kann, um die Monoether der Formel (Ib) zu erhalten; oder
+ um durch Einwirkung von Diphenylphosphorylazid in Gegenwart einer schwachen Base wie 1,8-Diazabicyclo[5.4.0]undec-7-en zu erhalten,
(E)-1,4-Bis(3-(diphenylphosphoryl)propyl)-1,4-dimethyltetrazen, ein Zwischenprodukt, das geeignet ist das Tetrazen der Formel (Ib), in der R3 = R4 = -CH₂N₃ zu erhalten, nach einem anderen Azidierungsverfahren, sowie das Tetrazen der Formel (Ib), in der R3 = R4 = **-**CH₂CN zu erhalten, durch Cyanierung, und sowie Gewinnung das Tetrazen der Formel (Ib), in der R3 = R4 = -CH₂N(CH₃)(NH₂) zu erhalten, durch nukleophile Substitution mit Monomethylhydrazin, oder
(E)-1-(3-(Diphenylphosphoryl)-4-(3-azidopropyl)-1,4-dimethyltetrazen, ein Zwischenprodukt, das geeignet ist, das Tetrazen der Formel (Ib) zu erhalten, in der R3 = -CH₂N₃ und R4 = -CH₂CN ist, durch Cyanierung, und das Tetrazen der Formel (Ib), in der R3 = -CH₂N₃ und R4 = -CH₂N(CH₃)(NH₂) zu erhalten, durch nukleophile Substitution mit Monomethylhydrazin.

8. Verfahren nach Anspruch 7, wobei 1-(3-Hydroxypropyl)-1-methylhydrazin durch Kondensation eines Überschusses an Monomethylhydrazin (MMH) mit 3-Chlorpropan-1-ol erhalten wird.

9. Verfahren nach Anspruch 7 oder 8, wobei 1-(3-Hydroxypropyl)-1-methylhydrazin mit KI₃ in wässriger Lösung oder mit Diod in Lösung in Ethanol oxidiert wird, vorteilhafterweise mit Diod in Lösung in Ethanol, und wobei 1-But-1-enyl-1-methylhydrazin mit Diod in Lösung in Diethylether oxidiert wird.

10. Verfahren nach Anspruch 2 oder 3, das zur Herstellung der β,γ-funktionalisierten 1,4-Dimethyltetrazene der Formel (Ic) Folgendes umfasst:
- Bereitstellung von 1-Azido-3-(1-methylhydrazinyl)propan-2-ol oder 1-Cyano-3-(1-methylhydrazinyl)propan-2-ol, dann
- durch Oxidation die Dimerisierung, wodurch das erwartete Tetrazen bzw. Diazido- oder Dicyanat entsteht.

11. Verfahren nach Anspruch 10, wobei das Bereitstellen des 1-Azido-3-(1-methylhydrazinyl)propan-2-ols oder des 1-Cyano-3-(1-methylhydrazinyl)propan-2-ols umfasst:
- die Öffnung des Epichlorhydrins durch Natriumnitrid bzw. Kaliumcyanid und
- die Kondensation des so erhaltenen Produkts mit Monomethylhydrazin (MMH), um das genannte 1-Azido-3-(1-methylhydrazinyl)propan-2-ol bzw. das genannte 1-Cyano-3-(1-methylhydrazinyl)propan-2-ol zu erhalten.

12. Verfahren nach Anspruch 10 oder 11, wobei 1-Azido-3-(1-methylhydrazinyl)propan-2-ol oder 1-Cyano-3-(1-methylhydrazinyl)propan-2-ol mit KI₃ in wässriger Lösung oxidiert werden.

13. Verfahren zur Herstellung von (E)-1,4-Bis(2-hydroxyethyl)-1,4-dimethyltetrazen, das umfasst:
- die Kondensation eines Überschusses an Monomethylhydrazin mit 2-Chlorethanol, um 1-(2-Hydroxyethyl)-1-methylhydrazin zu erhalten; dann
- die Dimerisierung des so erhaltenen Hydrazins durch Oxidation.

14. Verfahren nach Anspruch 13, wobei die Oxidation des 1-(2-Hydroxyethyl)-1-methylhydrazins durchgeführt wird a) mit Diiod in Lösung in einem organischen Lösungsmittel, insbesondere ausgewählt aus Ethanol und Diethylether und vorteilhafterweise bestehend aus Ethanol, oder b) mit KI₃ in wässriger Lösung oder c) mit Monochloramin; vorteilhafterweise wird die Oxidation mit Diiod in Lösung in Ethanol durchgeführt.

15. Zwischenprodukt, das zur Herstellung von mindestens einem der Tetrazene nach Anspruch 1 nützlich ist, wobei das Zwischenprodukt ausgewählt ist aus:
- 1-(2-Azidoethyl)-1-methylhydrazin;
- (E)-1,4-Bis(3-(diphenylphosphoryl)propyl)-1,4-dimethyltetrazen;
- (E)-1-(3-(Diphenylphosphoryl)-4-(3-azidopropyl)-1,4-dimethyltetrazen;
- 1-Azido-3-(1-methylhydrazinyl)propan-2-ol; und
- 1-Cyano-3-(1-methylhydrazinyl)propan-2-ol.

## Claims

1. A 1,4-Dimethyltetrazene selected from:
- β-functionalized 1,4-dimethyltetrazenes of formula (Ia):
**R1-CH₂-N(CH₃)-N=N-N(CH₃)-CH₂-R2** **(Ia)**
wherein
R1 = R2 = **-CH₂Cl, -CH₂N₃, -CH₂NH₂, -CH₂N(CH₃)(NH₂), -CH=CH₂,** or **-CH₂NCO** ; or
R1 and R2, which are identical, comprising an ether group, are selected from the following pairs:
R1 = R2 = **-CH₂O-CH₂-CH=CH₂,**
R1 = R2 = **-CH₂O-CH₂-C≡CH,** et
R1 = R2 = **-CH₂O-CH₂-C≡N** ;
- γ-functionalized 1,4-dimethyltetrazenes of formula (Ib):
**R3-CH₂-CH₂-N(CH₃)-N=N-N(CH₃)-CH₂-CH₂-R4** **(Ib)**
wherein
R3 = R4 = **-CH₂OH, -CH₂N₃, -CH₂NH₂, -CH₂N(CH₃)(NH₂), -CH=CH₂, -CH₂NCO, -CH₂CN** or **-CH₂ONO₂,** or
R3 = **-CH₂OH** and R4 = **-CH₂N₃,** or
R3 = **-CH₂CN** and R4 = **-CH₂N₃,** or
R3 = **-CH₂N₃** and R4 = **-CH₂N(CH₃)(NH₂),** or
R3 and R4, identical or different, at least one of which containing an ether group, are selected from the following pairs:
R3 = R4 = **-CH₂O-CH₂-CH=CH₂**
R3 = R4 = **-CH₂O-CH₂-C≡CH**
R3 = R4 = **-CH₂O-CH₂-C≡N**
R3 = **-CH₂O-CH₂-CH=CH₂** and R4 = **-CH₂N₃**
R3 = **-CH₂O-CH₂-C≡CH** and R4 = **-CH₂N₃,** and
R3 = **-CH₂O-CH₂-C≡N** and R4 = **-CH₂N₃;**
and
- β,γ-functionalized 1,4-dimethyltetrazenes of formula (Ic):
**R5-CHOH-CH₂-N(CH₃)-N=N-N(CH₃)-CH₂-CHOH-R5** **(Ic)**
wherein
R5 = **-CH₂N₃** or **-CH₂CN.**

2. A process for preparing 1,4-dimethyltetrazenes according to claim 1, which comprises:
- providing a precursor hydrazine, then,
- dimerizing it by oxidation.

3. The process of claim 2, wherein the oxidation of the precursor hydrazine is carried out a) with diiodine in an organic solvent, in particular selected from ethanol and diethyl ether and advantageously consisting of ethanol, or b) with an aqueous solution of KI₃, or c) with monochloramine.

4. The process of claim 2 or 3, which comprise, for the preparation of β-functionalized 1,4-dimethyltetrazenes of formula **(Ia):**
- providing a precursor hydrazine selected from 1-(2-chloroethyl)-1-methylhydrazine, in free and/or hydrochoride form, 1-(2-azidoethyl)-1-methylhydrazine, 1-(2-aminoethyl)-1-methylhydrazine, and 1-allyl-1-methylhydrazine, then obtaining by oxidation the corresponding dimer, a tetrazene of formula **(Ia)** in which, respectively, R1 = R2 = **-CH₂CI, -CH₂N₃, -CH₂NH₂** or **-CH=CH₂;** or
- obtaining (E)-1,4-bis(2-hydroxyethyl)-1,4-dimethyltetrazene and akylating the hydroxyl functions thereof, to obtain symmetrical diethers; and
- possibly, converting, by nucleophilic substitution with monomethylhydrazine, the tetrazene of formula **(Ia)** in which R1 = R2 = **-CH₂Cl** into the tetrazene of formula **(Ia)** in which R1 = R2 = **-CH₂N(CH₃)(NH₂)** or converting the tetrazene of formula **(Ia)** in which R1 = R2 = **-CH₂NH₂,** advantageously by reaction of its amine functions with 1,1'-carbonyldiimidazole, into the tetrazene of formua **(Ia)** in which R1 = R2 = **-CH₂NCO.**

5. The process of claim 4, wherein the 1-(2-azidoethyl)-1-methylhydrazine is obtained by azidation in an aqueous medium of 1-(2-chloroethyl)-1-methylhydrazine in free form.

6. The process of claim 4 or 5, wherein 1-(2-chloroethyl)-1-mehylhydrazine and 1-(2-azidoethyl)-1-methylhydrazine are oxidized with monochloramine, and wherein 1-allyl-1-methylhydrazine is oxidized with a solution of diiodine in diethyl ether.

7. The process of claim 2 or 3, which comprise, for the preparation of γ-functionalized 1,4-dimethyltetrazenes of formula **(Ib):**
- providing a precursor hydrazine selected from 1-(3-hydroxypropyl)-1-methylhydrazine, 1-(3-aminopropyl)-1-methylhydrazine and 1-but-1-enyl-1-methylhydrazine, then, by oxidation, obtaining the corresponding dimer, a tetrazene of formula **(Ib)** in which, respectively, R3 = R4 = **-CH₂OH, -CH₂NH₂** or **-CH=CH₂;**
- possibly, converting the tetrazene of formule **(Ib)** in which R3 = R4 = **-CH₂NH₂,** advantageously by reaction of its amine functions with 1,1'-carbonyl-dimidazole, into the tetrazene of formula **(Ib)** in which R3 = R4 = **-CH₂NCO** ;
- possibly, using the tetrazene of formula **(Ib)** in which R3 = R4 = **-CH₂OH** :
+ to obtain, by alkylation of its two hydroxyl functions, one of its symmetrical diethers of formula **(Ib)**, or
+ to obtain, by nitration, advantageously with nitronium tetrafluoroborate, the tetrazene of formula **(Ib)** in which R3 = R4 = **-CH₂ONO₂,** or
+ to obtain, by azidation, advantageously using tosyl azide in the presence of a weak base such as 1,8-diazabicyclo[5.4.0]undec-7-ene, the tetrazene of formula **(Ib)** in which R3 = R4 = **-CH₂N₃** and the tetrazene of formula **(Ib)** in which R3 **=-CH₂OH** and R4 = **-CH₂N₃,** if being possible for the hydroxyl function of the latter to be alkylated to obtain the monoethers of formula **(Ib) ;** or
+ to obtain, upon action of diphenylphosphoryl azide in the presence of a weak base such as 1,8-diazabicyclo[5.4.0]undec-7-ene,
*(E)*-1,4-bis(3-(diphenylphosphoryl)propyl)-1,4-dimethyltetrazene, an intermediate suitable for obtaining, by another azidation pathway, said tetrazene of formula **(Ib)** in which R3 = R4 = **-CH₂N₃,** as well as for obtaining, par cyanidation, of the tetrazene of formula **(Ib)** in which R3 = R4 = **-CH₂CN** and for obtaining, by nucleophilic substitution with monomethylhydrazine, the tetrazene of formula **(Ib)** in which R3 = R4 = **-CH₂N(CH₃)(NH₂),** or
*(E)*-1-(3-(diphenylphosphoryl)-4-(3-azidopropyl)-1,4-dimethyltetrazene, an intermediate suitable for obtaining, by cyanidation, the tetrazene of formula **(Ib)** in which R3 = **-CH₂N₃** and R4 = **-CH₂CN** and for obtaining, by nucleophilic substitution with monomethylhydrazine, the tetrazene of formula **(Ib)** in which R3 = **-CH₂N₃** and R4 = **-CH₂N(CH₃)(NH₂).**

8. The process of claim 7, wherein 1-(3-hydroxypropyl)-1-methylhydrazine is obtained by condensation of an excess of monomethylhydrazine (MMH) on 3-chloropropan-1-ol.

9. The process of claim 7 or 8, wherein 1-(3-hydroxypropyl)-1-methylhydrazine is oxidized with an aqueous solution of KI₃ or with a solution of diiodine in ethanol, advantageously with a solution of diiodine in ethanol, and wherein 1-but-1-enyl-1-methylhydrazine is oxidized with a solution of diiodine in diethyl ether.

10. The process of claim 2 or 3, which comprises, for the preparation of β,γ-functionalized 1,4-dimethyltetrazenes of formula **(Ic):**
- providing 1-azido-3-(1-methylhydrazinyl)propan-2-ol or 1-cyano-3-(1-methylhydrazinyl)propan-2-ol, then
- dimerization, by oxidation, to give the expected diazido or, respectively, dicyano tetrazene.

11. The process of claim 10, wherein providing 1-azido-3-(1-methylhydrazinyl)propan-2-ol or 1-cyano-3-(1-methylhydrazinyl)propan-2-ol comprises:
- opening of the epichlorhydrine, respectively by sodium nitride or potassium cyanide, and
- condensing the resulting product with monomethylhydrazine (MMH), for obtaining said 1-azido-3-(1-methylhydrazinyl)propan-2-ol, or, respectively, said 1-cyano-3-(1-methylhydrazinyl)propan-2-ol.

12. The process of claim 10 or 11, wherein 1-azido-3-(1-methylhydrazinyl)propan-2-ol or 1-cyano-3-(1-methylhydrazinyl)propan-2-ol are oxidized with an aqueous solution of KI₃.

13. A process for the preparation of *(E)-*1,4-bis(2-hydroxyethyl)-1,4-dimethyltetrazene, which comprises:
- condensing an excess of monomethylhydrazine on 2-chloroethanol to obtain 1-(2-hydroxyethyl)-l-methylhydrazine; then
- dimerizing the resulting hydrazine by oxidation.

14. The process of claim 13, wherein the oxidation of said 1-(2-hydroxyethyl)-1-methylhydrazine is carried out a) with a solution of diiodine in an organic solvent, in particular selected from ethanol and diethyl ether and advantageously consisting of ethanol, or b) with an aqueous solution of KI₃, or c) with monochloramine; advantageously the oxidation is carried out with a solution of diiodine in ethanol.

15. An intermediate useful for the preparation of at least one of the tetrazenes according to claim 1, said intermediate being selected from:
- 1-(2-azidoethyl)-1-methylhydrazine ;
- *(E)*-1,4-bis(3-(diphenylphosphoryl)propyl)-1,4-dimethyltetrazene ;
- *(E)*-1-(3-(diphenylphosphoryl)-4-(3-azidopropyl)-1,4-dimethyltetrazene ;
- 1-azido-3-(1-methylhydrazinyl)propan-2-ol ; and
- 1-cyano-3-(1-methylhydrazinyl)propan-2-ol.
